(19) Europäisches Patentamt
European Patent Office
Office européen des brevets

(11) Publication number: 0 141 962
A1

(12) EUROPEAN PATENT APPLICATION

(21) Application number: 84110835.0

(22) Date of filing: 11.09.84

(51) Int. Cl.⁴: C 07 D 491/107, C 07 D 311/82, G 03 G 5/12

(30) Priority: 15.09.83 US 532420

(43) Date of publication of application: 22.05.85 Bulletin 85/21

(84) Designated Contracting States: CH DE GB LI

(71) Applicant: THE HILTON - DAVIS CHEMICAL COMPANY, 2235 Langdon Farm Road, Cincinnati Ohio (US)

(72) Inventor: Schmidt, Paul Joseph, 3590 Concerto Drive, Sharonville Ohio (US)
Inventor: Hung, William Mo-Wei, 9176 Millcliff Drive, Cincinnati Ohio (US)

(74) Representative: Baillie, Iain Cameron et al, c/o Ladas & Parry Isartorplatz 5, D-8000 München 2 (DE)

(54) **Hydrazine derivatives of fluorans and use thereof in electrochromic recording systems.**

(57) This invention relates to spiro[isoindole-xanthene]-3-ones, spiro[phthalazine-xanthene]-4-ones, and xanthene-3-ones useful as color-forming substances, particularly in electrochromic recording systems, which are prepared by the interaction of a fluoran with a substituted or unsubstituted hydrazine.

EP 0 141 962 A1

## BACKGROUND OF THE INVENTION

### (a) Field of the Invention

This invention relates to novel compounds classified in the field of organic chemistry as spiro[isoindole-xanthene]-3-ones, spiro[phthalazine-xanthene]-4-ones and xanthene-3-ones useful as color-forming substances, particularly in the art of electrochromic recording; to electrochromic recording systems containing said compounds; and to processes for preparing said compounds.

### (b) Information Disclosure Statement

Several classes of organic compounds of widely diverse structural types are known to be useful as colorless precursors for electrochromic recording. Among the more important classes, there may be named leuco-type dyestuffs such as: phthalides, for example, crystal violet lactone, Malachite green lactone; fluorans, for example, 3-diethylamino-5,7-dimethylfluoran; and indolinobenzospiropyrans, for example, 1,3,3-trimethyl-6'-chloro-8'-methoxyindolinobenzospiropyran. Also utilized as colorless precursors for electrochromic recording, either alone or in admixture with the leuco compounds indicated above, are substances known as redox indicators. The redox indicator which becomes colored *in situ* in the electrochromic recording process also is generally a leuco compound. Among the

types of compounds which are applicable as redox indicators are phenothiazines, for example, leuco methylene blue and benzoyl leuco methylene blue. Other specific indicators are Leucoethyl Nile Blue, Leucomethyl Capryl Blue and Leucosafranine T. Typical of the many such electrochromic recording systems taught in the prior art are those described in U.S. Patents 3,726,769, 3,871,972, 3,864,684, 4,017,366, 4,133,933, and Reissue Patent Re. 29,427 which issued April 10, 1973, March 18, 1975, February 4, 1975, April 12, 1977, January 9, 1979, and October 4, 1977, respectively. The methods for electrochromic recording taught in the prior art have many variations. Basically, one or both sides of a sheet of paper is coated or treated with a coating formulation containing at least one colorless color-forming (leuco) compound. Electrical current is then selectively applied to the coated side of the paper by some means, for example, a stylus or a printing head to which an electrical potential can be applied. The application of the current causes an electrochromic reaction involving the leuco compound to produce a visible image corresponding to the design traced by the stylus or that of the printing head.

The following items to date appear to constitute the most relevant prior art with regard to the instant invention.

Kuzuya et al. in Chemical Pharmaceutical Bulletin 1980, 28(12), 3561-9 (C.A. 94:141182 p) describe the preparation and physical characteristics of 3',6'-bis(diethylamino)-spiro-[1H-isoindole-1,9'-(9H)-xanthene]-3(2H)-one having the structural formula

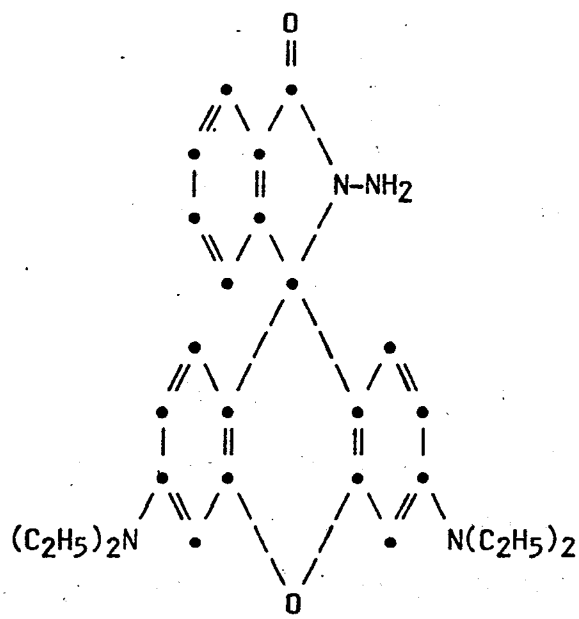

This compound was reportedly prepared by heating Rhodamine with hydrazine hydrate. No utility is taught for the compound. The compound was prepared as part of a research program assessing the influence of structural change on the formation of dihydrophthalazinones.

German Patent 85,242, granted December 30, 1895, discloses a compounds having the formula

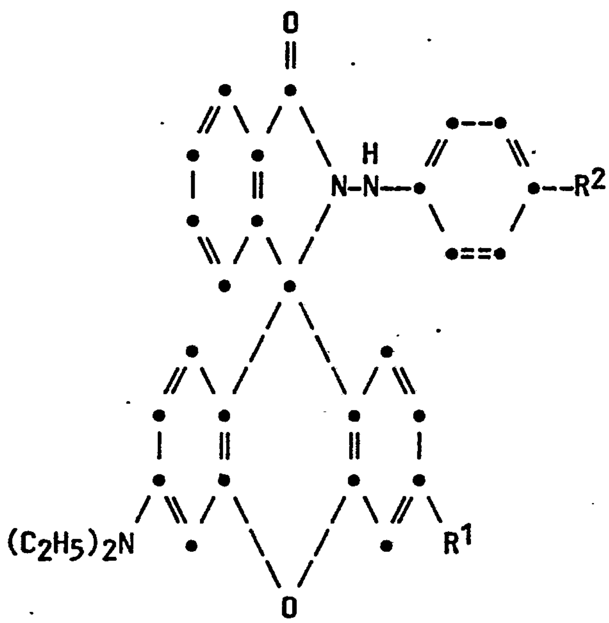

in which $R^1$ is hydrogen or diethylamino and $R^2$ is hydrogen or methyl. These compounds were reportedly prepared by heating the corresponding Rhodamine with the corresponding phenyl-substituted hydrazine. The compounds are alleged to form intense blue-red colors in the presence of acids.

Japanese Patent Publication 51,054,623, published May 13, 1976, discloses phenylhydrazino lactams having the structural formula

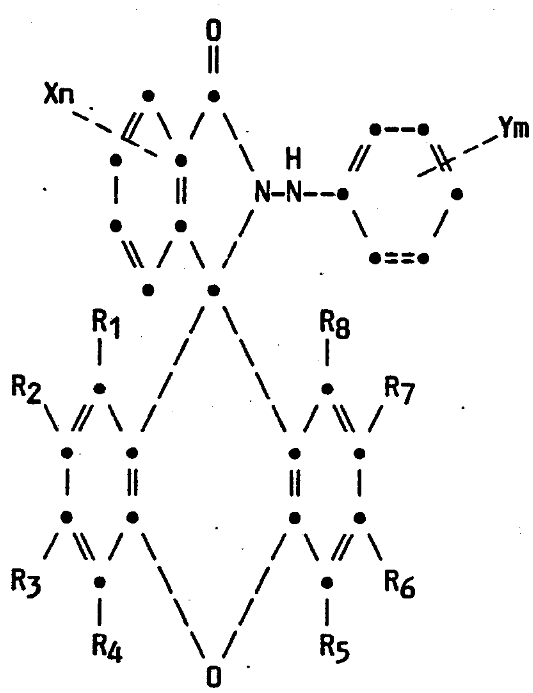

in which $R_1$ is hydrogen; $R_2$ is hydrogen, halogen or lower alkyl; $R_3$ is alkoxy, lower monoalkylamino, lower dialkylamino, cyclohexylamino, N-cyclohexyl-N-lower alkylamino, substituted or non-substituted arylamino, substituted or non-substituted N-aryl-N-lower alkylamino, substituted or non-substituted N-aryl-N-benzylamino, substituted or non-substituted benzylamino, morpholino, pyrrolidino, or substituted or non-substituted piperidino; $R_4$ is hydrogen, halogen or lower alkyl; $R_5$ is hydrogen, halogen, lower alkyl, alkoxy or acylamino; $R_6$ is hydrogen, halogen, lower alkyl or alkoxy; $R_7$ is hydrogen, halogen, alkyl, alkoxy, phenyl, substituted or non-substituted dibenzylamino, substituted or non-substituted arylamino, substituted

or non-substituted-N-aryl-N-alkylamino, substituted or non-substituted diphenylmethylamino, morpholino, pyrrolidino, or substituted or non-substituted piperidino; $R_8$ is hydrogen, lower alkyl or alkoxy; X is hydrogen, halogen or lower alkyl; Y is hydrogen, halogen or alkyl; n is a number selected from 1 to 4; and m is a number from 1 to 5. The lactams were reportedly prepared by reacting the appropriate fluoran with the appropriate phenylhydrazine. The lactams are disclosed as colorants for heat-sensitive recording material, circular telegraphic recording material and high-frequency recording material.

U.S. Patent 3,185,696, issued May 25, 1965, discloses and claims compounds having the formula

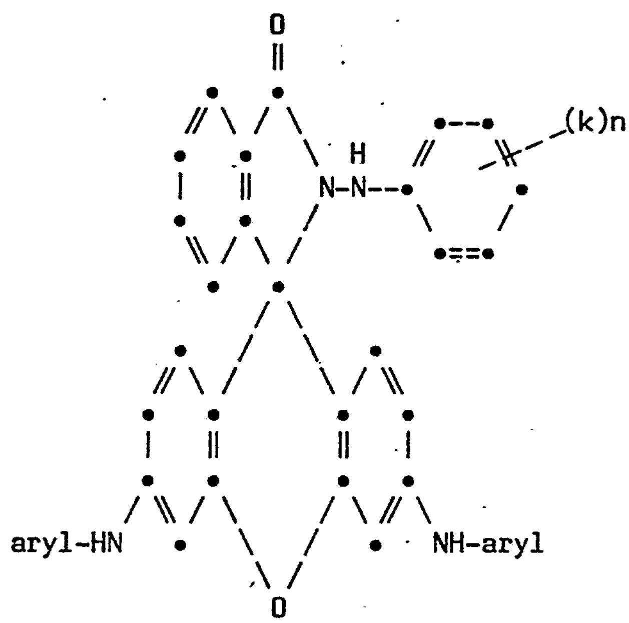

in which aryl is a member selected from the group consisting

of cyclohexylphenyl, naphthyl and 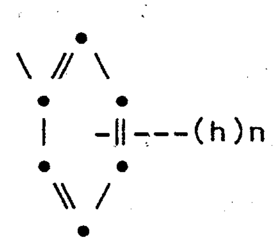

in which h is a member of the group consisting of hydrogen, lower alkyl, hydroxy, lower alkoxy and halogen; k is a member selected from the group consisting of hydrogen, lower alkyl, hydroxy, lower alkoxy, nitro, halogen and cyano; and n is an integer from 1 to 4. The compounds were reportedly prepared by heating the appropriate fluoran with the appropriate phenyl-substituted hydrazine. The compounds are disclosed as colorless solids which are capable of producing dye color immediately upon intimate contact with a color initiator such as clay, a zeolite, a phenol and/or a heteropoly acid.

U.S. Patent 4,017,366, issued April 12, 1977, discloses and claims a method of printing on thermographic recording paper according to which the heat for causing the imaging color change in the thermographic coating is generated by means of current in a portion of the coating dampened by a thin film of a conductive salt solution which is dispersed on the thermographic coating. The current is generated by a printing head having spaced electrodes in contact with the dampened portion of the coating.

SUMMARY OF THE INVENTION

In its first composition of matter aspect, the invention relates to certain spiro[1H-isoindole-1,9'-[9H]-xanthene]-3(2H)-ones useful as colorless precursors in electrochromic recording systems.

In its second composition of matter aspect, the invention relates to certain 2,3-dihydro-spiro[phthalazine-1(4H),9'-[9H]-xanthene]-4-ones useful as colorless precursors in electrochromic recording systems.

In its third composition of matter aspect, the invention relates to certain 3H-xanthene-3-ones useful as colorless precursors in electrochromic recording systems.

The present invention provides in its first article of manufacture aspect, a substrate for use in electrochromic recording systems comprising a support sheet containing as a color-forming substance a spiro[1H-isoindole-1,9'-[9]-xanthene]-3(2H)-one.

The present invention provides in its second article of manufacture aspect, a substrate for use in electrochromic recording systems comprising a support sheet containing as a color-forming substance a 2,3-dihydro-spiro-[phthalazine-1(4H),9'-[9H]-xanthene]-4-one.

The present invention provides in its third article of manufacture aspect, a substrate for use in electrochromic recording systems comprising a support sheet containing as a color-forming substance a 3H-xanthene-3-one.

In its first process aspect, the invention relates to a process for producing a compound selected from the group consisting of spiro[1H-isoindole-1,9'-[9H]-xanthene]-3(2H)-ones and 2,3-dihydro-spiro[phthalazine-1(4H),9'-[9H]-xanthene]-4-ones and mixtures thereof which comprises interacting the corresponding fluoran with the corresponding hydrazine.

In its second process aspect, the invention relates to a process for producing a compound selected from the group consisting of 3,6-dihydroxy-spiro[1H-isoindole-1,9'-[9H]-xanthene]-3(2H)-ones and 6-hydroxy-3H-xanthene-3-ones and mixtures thereof which comprises interacting the corresponding fluoran with the corresponding hydrazine.

## DETAILED DESCRIPTION INCLUSIVE OF THE PREFERRED EMBODIMENTS

More specifically, this invention in its first composition of matter aspect resides in the novel 2-($R^6$-amino)-2'-R-3'-$R^1$-4'-$R^2$-5'-$R^3$-6'-$R^4$-7'-$R^5$-5/6-Y-spiro[1H-isoindole-1,9'-[9H]-xanthene]-3(2H)-one having the structural formula

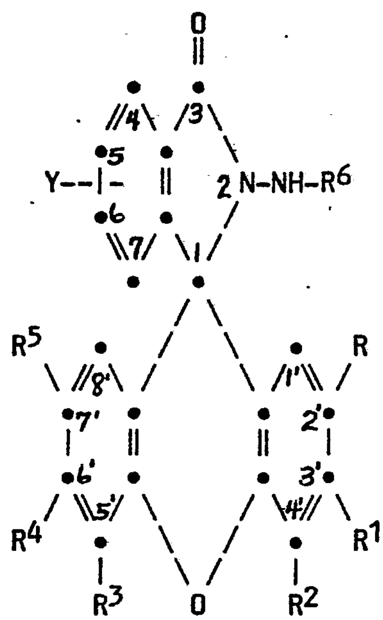

Formula I

wherein: R represents hydrogen, halogen, non-tertiary $C_1$ to $C_4$ alkyl or $-N(R^7)(R^8)$ in which $R^7$ represents hydrogen, non-tertiary $C_1$ to $C_{12}$ alkyl, acetyl, benzyl or benzyl substituted in the benzene ring by one or two of halo, nitro, non-tertiary $C_1$ to $C_4$ alkyl or non-tertiary $C_1$ to $C_4$ alkoxy, and $R^8$ represents hydrogen, non-tertiary $C_1$ to $C_{12}$ alkyl, phenyl, phenyl substituted by one or two of halo, nitro, non-tertiary $C_1$ to $C_4$ alkyl or non-tertiary $C_1$ to $C_4$ alkoxy, benzyl, benzyl substituted in the benzene ring by one or two of halo, nitro, non-tertiary $C_1$ to $C_4$ alkyl or non-tertiary $C_1$ to $C_4$ alkoxy, the group

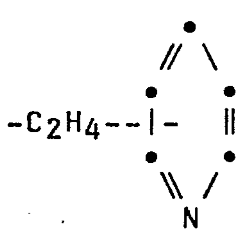 or $R^7$ and $R^8$, taken together with the

nitrogen, represent pyrrolyl; $R^1$ represents hydrogen, hydroxy or non-tertiary $C_1$ to $C_4$ alkyl; $R^2$, $R^3$ and $R^5$ independently represent hydrogen or halo; $R^4$ represents hydroxy or $-N(R^9)(R^{10})$ in which $R^9$ represents non-tertiary $C_1$ to $C_4$ alkyl, cyclohexyl, phenyl, phenyl substituted by one or two of halo, nitro, non-tertiary $C_1$ to $C_4$ alkyl or non-tertiary $C_1$ to $C_4$ alkoxy, benzyl or benzyl substituted in the benzene ring by one or two of halo, nitro, non-tertiary $C_1$ to $C_4$ alkyl or non-tertiary $C_1$ to $C_4$ alkoxy; $R^{10}$ represents non-tertiary $C_1$ to $C_4$ alkyl, benzyl or benzyl substituted in the benzene ring by one or two of halo, nitro, non-tertiary $C_1$ to $C_4$ alkyl or non-tertiary $C_1$ to $C_4$ alkoxy; $R^9$ and $R^{10}$ taken together with the nitrogen represent pyrrolidyl or piperidyl; $R^6$ represents hydrogen, phenyl, phenyl substituted by one or two of non-tertiary $C_1$ to $C_4$ alkyl, non-tertiary $C_1$ to $C_4$ alkoxy, halo, nitro or $\overset{O}{\overset{\|}{C}}X$ in which X is non-tertiary $C_1$ to $C_4$ alkyl, non-tertiary $C_1$ to $C_4$ alkoxy, $NHNH_2$ or $\overset{O}{\overset{\|}{C}}NHNH_2$; and Y represents hydrogen, nitro, amino, one to four halo or $\overset{O}{\overset{\|}{C}}Z$ in which Z represents $OR^{11}$ wherein $R^{11}$ represents hydrogen, non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy.

Preferred compounds within the ambit of the first composition of matter aspect are the novel 2-($R^6$-amino-2'-R-3'-$R^1$-6'-(N-$R^9$-N-$R^{10}$-amino)-5/6-Y-spiro[1H-isoindole-1,9'-[9H]-xanthene]-3(2H)-ones of Formula I wherein $R^2$, $R^3$ and $R^5$ are hydrogen and $R^4$ is (N-$R^9$-N-$R^{10}$-amino) having the structural formula

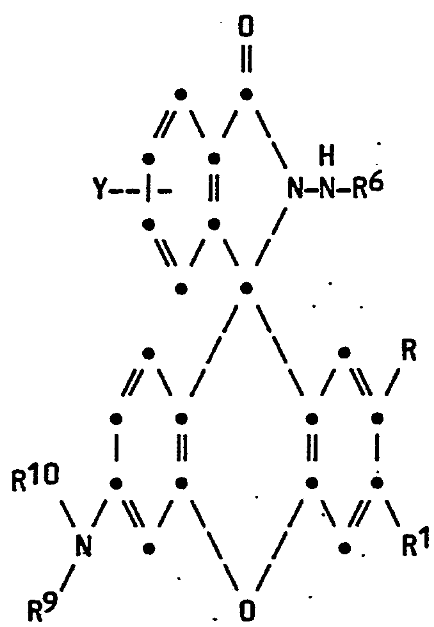

Formula II

wherein R, $R^1$, $R^6$, $R^9$, $R^{10}$ and Y have the same respective meanings given in Formula I; the novel 2-($R^6$-amino)-2'-(N-$R^7$-N-$R^8$-amino)-3'-$R^1$-6'-(N-$R^9$-N-$R^{10}$-amino)-5/6-Y-spiro[1H-isoindole-1,9'-[9H]-xanthene]-3(2H)-ones of Formula II wherein R is (N-$R^7$-N-$R^8$-amino) having the structural formula

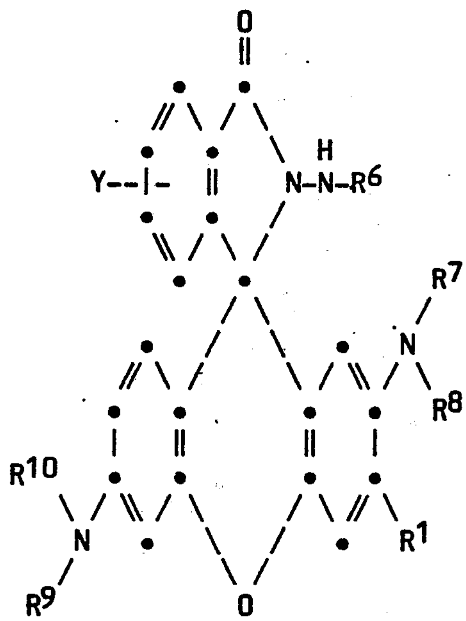

Formula III

in which $R^1$, $R^6$, $R^7$, $R^8$, $R^9$, $R^{10}$ and Y have the same respective meanings given in Formula II; and the novel 2-($R^6$-amino)-2'-(N-$R^7$-N-$R^8$-amino)-2'-R-3'-hydroxy-4'-$R^2$-5'-$R^3$-6'-hydroxy-7'-$R^5$-5/6-Y-spiro[1H-isoindole-1,9'-[9H]-xanthene]-3(2H)-ones of Formula I wherein $R^1$ and $R^4$ are each hydroxy having the structural formula

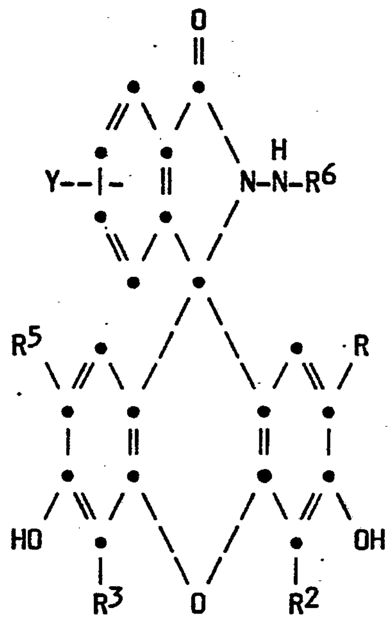

Formula IV

wherein R, $R^2$, $R^3$, $R^5$, $R^6$ and Y have the same respective meanings given in Formula I.

In the second composition of matter aspect, the invention sought to be patented resides in the novel 2-$R^6$-2'-R-3'-$R^1$-4'-$R^2$-5'-$R^3$-6'-$R^4$-7'-$R^5$-6/7-Y-2,3-dihydro-spiro-[phthalazine-1(4H),9'-[9H]-xanthene]-4-one having the structural formula

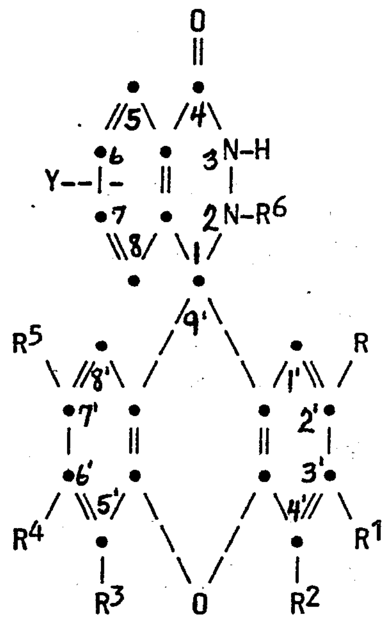

Formula V

wherein: R represents hydrogen, halogen, non-tertiary $C_1$ to $C_4$ alkyl or $-N(R^7)(R^8)$ in which $R^7$ represents hydrogen, non-tertiary $C_1$ to $C_{12}$ alkyl, acetyl, benzyl or benzyl substituted in the benzene ring by one or two of halo, nitro, non-tertiary $C_1$ to $C_4$ alkyl or non-tertiary $C_1$ to $C_4$ alkoxy, and $R^8$ represents hydrogen, non-tertiary $C_1$ to $C_{12}$ alkyl, phenyl, phenyl substituted in the benzene ring by one or two of halo, nitro, non-tertiary $C_1$ to $C_4$ alkyl or non-tertiary $C_1$ to $C_4$ alkoxy, benzyl, benzyl substituted in the benzene ring by one or two of halo, nitro, non-tertiary $C_1$ to $C_4$ alkyl or non-tertiary $C_1$ to $C_4$ alkoxy, the group

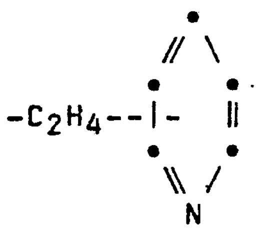 or $R^7$ and $R^8$, taken together with the nitrogen, represent pyrrolyl; $R^1$ represents hydrogen, hydroxy or non-tertiary $C_1$ to $C_4$ alkyl; $R^2$, $R^3$ and $R^5$ independently represent hydrogen or halo; $R^4$ represents hydroxy or $-N(R^9)(R^{10})$ in which $R^9$ represents non-tertiary $C_1$ to $C_4$ alkyl, cyclohexyl, phenyl, phenyl substituted by one or two of halo, nitro, non-tertiary $C_1$ to $C_4$ alkyl or non-tertiary $C_1$ to $C_4$ alkoxy, benzyl or benzyl substituted in the benzene ring by one or two of halo, nitro, non-tertiary $C_1$ to $C_4$ alkyl or non-tertiary $C_1$ to $C_4$ alkoxy; $R^{10}$ represents non-tertiary $C_1$ to $C_4$ alkyl, benzyl or benzyl substituted in the benzene ring by one or two of halo, nitro, non-tertiary $C_1$ to $C_4$ alkyl or non-tertiary $C_1$ to $C_4$ alkoxy; $R^9$ and $R^{10}$ taken together with the nitrogen represent pyrrolidyl or piperidyl; $R^6$ represents hydrogen, phenyl, phenyl substituted by one or two of non-tertiary $C_1$ to $C_4$ alkyl, non-tertiary $C_1$ to $C_4$ alkoxy, halo, nitro or $\overset{O}{\overset{\|}{C}}X$ in which X is non-tertiary $C_1$ to $C_4$ alkyl, non-tertiary $C_1$ to $C_4$ alkoxy, $NHNH_2$ or $\overset{O}{\overset{\|}{C}}NHNH_2$; and Y represents hydrogen, nitro, amino, one to four halo or $\overset{O}{\overset{\|}{C}}Z$ in which Z represents $OR^{11}$ wherein $R^{11}$ represents hydrogen, non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy.

Preferred compounds within the ambit of the second composition of matter aspect are the novel 2-$R^6$-2'-R-3'-$R^1$-6'-(N-$R^9$-N-$R^{10}$-amino)-6/7-Y-2,3-dihydro-spiro[phthalazine-1(4H),9'-[9]-xanthene]-4-ones of Formula V wherein $R^2$, $R^3$ and $R^5$ are hydrogen and $R^4$ is (N-$R^9$-N-$R^{10}$-amino) having the structural formula

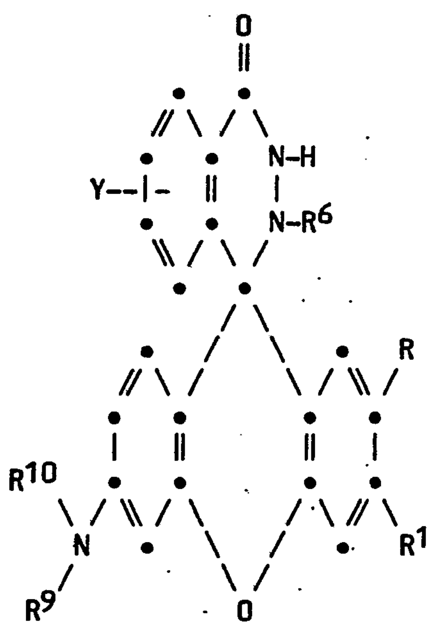

Formula VI

wherein R, $R^1$, $R^6$, $R^9$, $R^{10}$ and Y have the same respective meanings given in Formula V; and the novel 2-$R^6$-2'-(N-$R^7$-N-$R^8$-amino)-3'-$R^1$-6'-(N-$R^9$-N-$R^{10}$-amino)-6/7-Y-2,3-dihydro-spiro[phthalazine-1(4H),9'-[9]-xanthene]-4-ones of Formula VI wherein R is (N-$R^7$-N-$R^8$-amino) having the structural formula

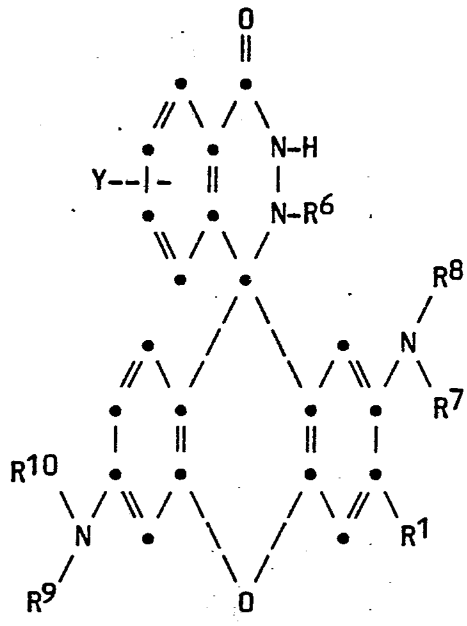

Formula VII

wherein $R^1$, $R^6$, $R^7$, $R^8$, $R^9$, $R^{10}$ and Y have the same respective meanings given in Formula VI.

In the third composition of matter aspect, the invention sought to be patented resides in the novel 2-R-4-$R^2$-5-$R^3$-6-hydroxy-7-$R^5$-9-[2-(N-$R^6$-hydrazocarbonyl)-Y-phenyl]-3H-xanthene-3-ones having the structural formula

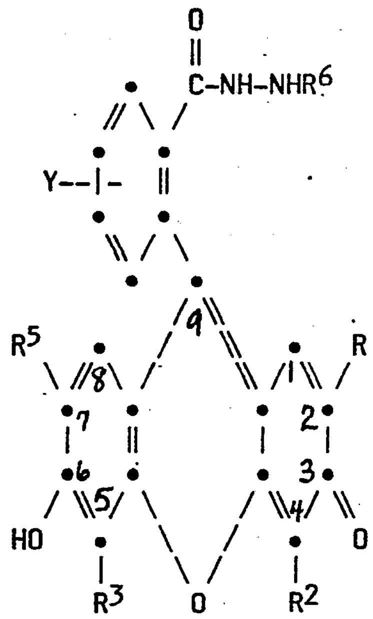

Formula VIII

wherein R, $R^2$, $R^3$ and $R^5$ independently represent hydrogen or halo; $R^6$ represents hydrogen, non-tertiary $C_1$ to $C_4$ alkyl, phenyl, phenyl substituted by one or two of non-tertiary $C_1$ to $C_4$ alkyl, non-tertiary $C_1$ to $C_4$ alkoxy, halo, nitro or amino or $\overset{O}{\overset{\|}{C}}X$ in which X is non-tertiary $C_1$ to $C_4$ alkyl, non-tertiary $C_1$ to $C_4$ alkoxy, $NHNH_2$ or $\overset{O}{\overset{\|}{C}}NHNH_2$; and Y represents hydrogen, nitro, amino, one to four of halo, or $\overset{O}{\overset{\|}{C}}Z$ in which Z represents $OR^{11}$ wherein $R^{11}$ represents hydrogen, a non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy.

Preferred compounds within the ambit of the third composition of matter aspect are the novel 2-R-4-$R^2$-5-$R^3$-6-hydroxy-7-$R^5$-9-(2-hydrazocarbonyl-Y-phenyl)-3H-xanthene-3-ones of Formula VIII having the structural formula

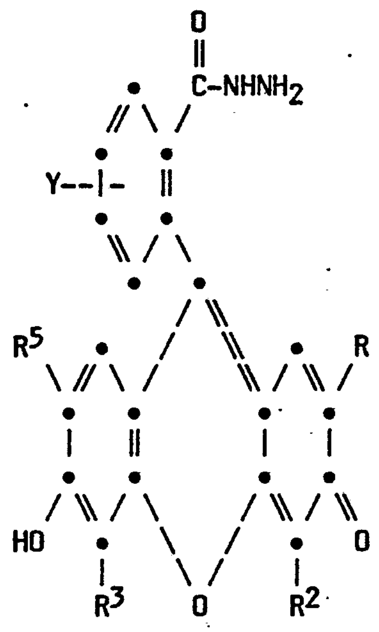

Formula IX

in which R, $R^2$, $R^3$, $R^4$, $R^5$ and Y have the same respective meanings given in Formula VIII.

In the first of its article of manufacture aspects, the invention sought to be patented resides in a substrate for use in electrochromic recording comprising a support sheet containing as a color-forming substance a 2-($R^6$-amino)-2'-R-3'-$R^1$-4'-$R^2$-5'-$R^3$-6'-$R^4$-7'-$R^5$-5/6-Y-spiro[1H-isoindole-1,9'-[9H]-xanthene]-3(2H)-one according to Formula I wherein R, $R^1$, $R^2$, $R^3$, $R^4$, $R^5$, $R^6$ and Y have the same respective meanings given relative to Formula I.

Preferred embodiments within the ambit of this first article of manufacture aspect are the substrates for use in electrochromic recording comprising a support sheet containing as a color-forming substance a 2-($R^6$-amino)-2'-R-3'-$R^1$-6'-(N-$R^9$-N-$R^{10}$-amino)-5/6-Y-spiro[1H-isoindole-1,9'-[9H]-xanthene]-3(2H)-one of Formula II wherein R, $R^1$, $R^6$, $R^9$, $R^{10}$ and Y have the same respective meanings given in Formula II; a 2-($R^6$-amino)-2'-(N-$R^7$-N-$R^8$-amino)-3'-$R^1$-6'-(N-$R^9$-N-$R^{10}$-amino)-5/6-Y-spiro[1H-isoindole-1,9'-[9H]-xanthene]-3(2H)-one of Formula III wherein R, $R^6$, $R^7$, $R^8$, $R^9$, $R^{10}$ and Y have the same respective meanings given in Formula III; or a 2-($R^6$-amino)-2'-R-3',6'-dihydroxy-4'-$R^2$-5'-$R^3$-7'-$R^5$-5/6-Y-spiro[1H-isoindole-1,9'-[9H]-xanthene]-3(2H)-one of Formula IV wherein R, $R^2$, $R^3$, $R^5$, $R^6$ and Y have the same respective meanings given in Formula IV.

In the second of its article of manufacture aspects, the invention sought to be patented resides in a substrate for use in electrochromic recording comprising a support sheet containing as a color-forming substance a 3-$R^6$-2'-R-3'-$R^1$-4'-$R^2$-5'-$R^3$-6'-$R^4$-7'-$R^5$-6/7-Y-2,3-dihydro-spiro[phthalazine-1(4H),9'-[9H]-xanthene]-4-one according to Formula V wherein R, $R^1$, $R^2$, $R^3$, $R^4$, $R^5$, $R^6$ and Y have the same respective meanings given relative to Formula V.

Within the ambit of this second article of manufacture aspect, preferred substrates for use in electrochromic recording are those comprising a support sheet containing as a color-forming substance a 3-$R^6$-2'-R-3'-$R^1$-6'-(N-$R^9$-N-$R^{10}$-amino)-6/7-Y-2,3-dihydro-spiro[phthalazine-1(4H),9'-[9H]-xanthene]-4-one of Formula VI wherein R, $R^1$, $R^6$, $R^9$, $R^{10}$ and Y have the same respective meanings given in Formula VI; and particularly preferred are support sheets containing as color-forming substances the 3-$R^6$-2'-(N-$R^7$-N-$R^8$-amino)-3-$R^1$-6-(N-$R^9$-N-$R^{10}$-amino)-6/7-Y-2,3-dihydro-spiro-[phthalazine-1(4H),9'-[9H]-xanthene]-4-ones of Formula VII wherein R, $R^6$, $R^7$, $R^8$, $R^9$, $R^{10}$ and Y have the same respective meanings given in Formula VII.

In the third of its article of manufacture aspects, the invention sought to be patented resides in a substrate for use in electrochromic recording comprising a support sheet containing as a color-forming substance a 2-R-4-$R^2$-5-$R^3$-6-hydroxy-7-$R^5$-9-[2-(N-$R^6$-hydrazocarbonyl)-Y-phenyl]-3H-xanthene-3-one according to Formula VIII wherein R, $R^2$, $R^3$, $R^4$, $R^5$, $R^6$ and Y have the same respective meanings given relative to Formula VIII.

Preferred embodiments within the ambit of this third article of manufacture aspect are the substrates for use in electrochromic recording comprising a support sheet containing as a color-forming substance a 2-R-4-$R^2$-5-$R^3$-6-hydroxy-7-$R^5$-9-(2-hydrazinocarbonyl-Y-phenyl)-3H-xanthene-3-one of Formula IX wherein R, $R^2$, $R^3$, $R^5$ and Y have the same respective meanings given in Formula IX.

In its first process aspect, the invention sought to be patented resides in the process for preparing a compound selected from the group having the structural formula

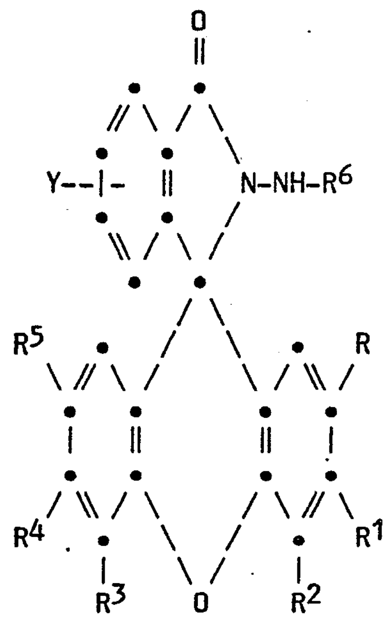

Formula I

and the structural formula

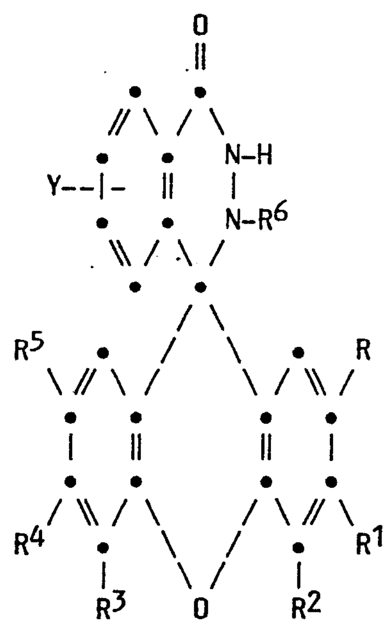

Formula V

and mixtures thereof which comprises interacting a compound having the structural formula

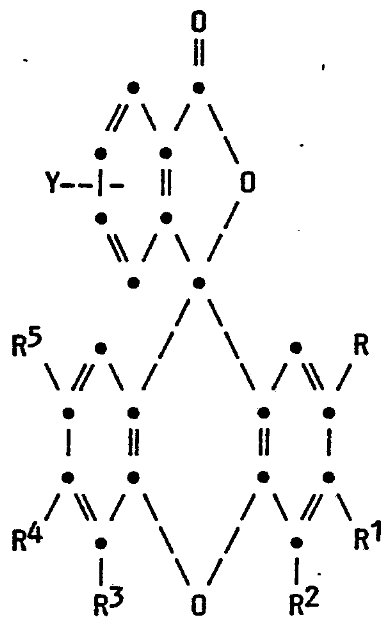

Formula X

with a compound of the structural formula

$R^6NHNH_2$

Formula XI

wherein in Formulas I, V, X and XI, R represents hydrogen, halogen, non-tertiary $C_1$ to $C_4$ alkyl or $-N(R^7)(R^8)$ in which $R^7$ represents hydrogen, non-tertiary $C_1$ to $C_{12}$ alkyl, acetyl, benzyl or benzyl substituted in the benzene ring by one or two of halo, nitro, non-tertiary $C_1$ to $C_4$ alkyl or non-tertiary $C_1$ to $C_4$ alkoxy, and $R^8$ represents hydrogen, non-tertiary $C_1$ to $C_{12}$ alkyl, phenyl, phenyl substituted by one or two of halo, nitro, non-tertiary $C_1$ to $C_4$ alkyl or non-tertiary $C_1$ to $C_4$ alkoxy, benzyl, benzyl substituted in the benzene ring by one or two of halo, nitro, non-tertiary $C_1$ to $C_4$ alkyl or non-tertiary $C_1$ to $C_4$ alkoxy, the group

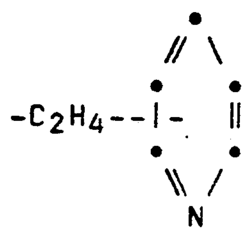 or $R^7$ and $R^8$, taken together with the nitrogen, represent pyrrolyl; $R^1$ represents hydrogen, hydroxy or non-tertiary $C_1$ to $C_4$ alkyl; $R^2$, $R^3$ and $R^5$ independently represent hydrogen or halo; $R^4$ represents hydroxy or $-N(R^9)(R^{10})$ in which $R^9$ represents non-tertiary $C_1$ to $C_4$ alkyl, cyclohexyl, phenyl, phenyl substituted by one or two of halo, nitro, non-tertiary $C_1$ to $C_4$ alkyl or non-tertiary $C_1$ to $C_4$ alkoxy, benzyl or benzyl substituted in the benzene ring by one or two of halo, nitro, non-tertiary $C_1$ to $C_4$ alkyl or non-tertiary $C_1$ to $C_4$ alkoxy; $R^{10}$ represents non-tertiary $C_1$ to $C_4$ alkyl, benzyl or benzyl substituted in the benzene ring by one or two of halo, nitro, non-tertiary $C_1$ to $C_4$ alkyl or non-tertiary $C_1$ to $C_4$ alkoxy; $R^9$ and $R^{10}$ taken together with the nitrogen represent pyrrolidyl or piperidyl; $R^6$ represents hydrogen, phenyl, phenyl substituted by one or two of non-tertiary $C_1$ to $C_4$ alkyl, non-tertiary $C_1$ to $C_4$ alkoxy, halo, nitro or $\overset{O}{\overset{\|}{C}}X$ in which X is non-tertiary $C_1$ to $C_4$ alkyl, non-tertiary $C_1$ to $C_4$ alkoxy, $NHNH_2$ or $\overset{O}{\overset{\|}{C}}NHNH_2$; and Y represents hydrogen, nitro, amino, one to four halo, or $\overset{O}{\overset{\|}{C}}Z$ in which Z represents $OR^{11}$ wherein $R^{11}$ represents hydrogen, a non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy.

In its second process aspect, the invention sought to be patented resides in the process for preparing a compound selected from the group having the structural formula

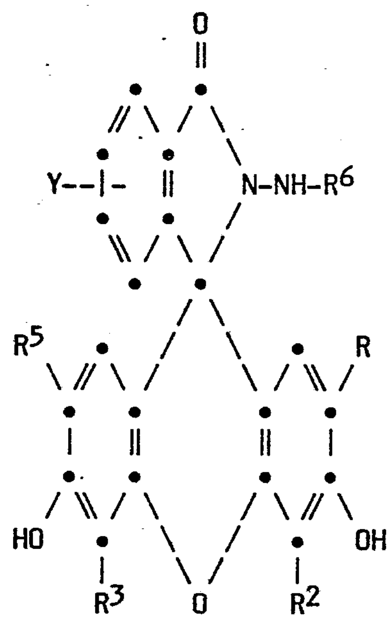

Formula IV

and the structural formula

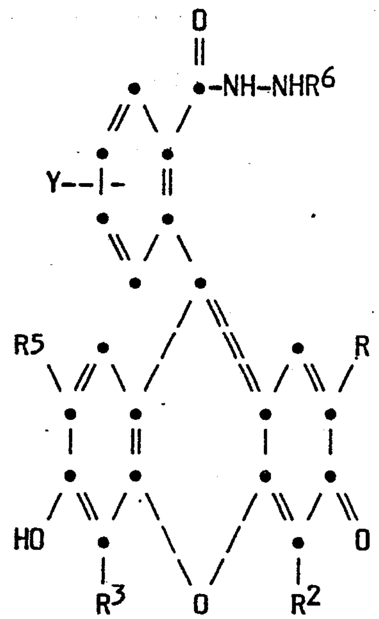

Formula XII

and mixtures thereof which comprises interacting a compound having the structural formula

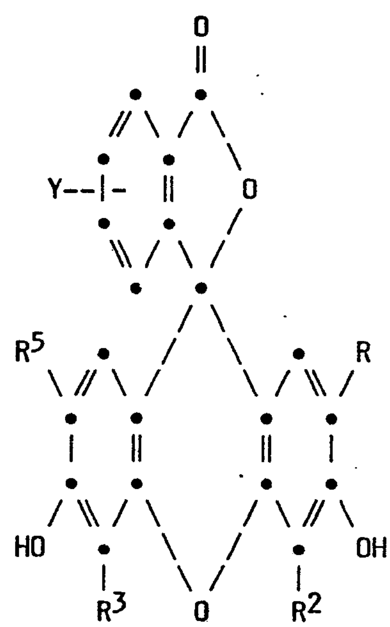

Formula XIII

with a compound of the structural formula

$$R^6NHNH_2$$

<u>Formula XI</u>

wherein in Formulas IV, XI, XII, and XIII, R, $R^2$, $R^3$ and $R^5$ each independently represent hydrogen or halo; $R^6$ represents hydrogen, phenyl, phenyl substituted by one or two of non-tertiary $C_1$ to $C_4$ alkyl, non-tertiary $C_1$ to $C_4$ alkoxy, halo, nitro or $\overset{O}{\overset{\|}{C}}X$ in which X is non-tertiary $C_1$ to $C_4$ alkyl, non-tertiary $C_1$ to $C_4$ alkoxy, $NHNH_2$ or $\overset{O}{\overset{\|}{C}}NHNH_2$ and Y represents hydrogen, nitro, one to four halo or $\overset{O}{\overset{\|}{C}}Z$ in which Z represents $OR^{11}$ wherein $R^{11}$ represents hydrogen, a non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy.

As used herein the term "halo" includes chloro, fluoro, bromo and iodo. Chloro is the preferred halo substituent because of the relatively low cost and ease of preparation of the required chloro-substituted intermediates and because the other halogens offer no particular advantages over chloro. However, the other above-named halo substituents are also satisfactory.

The term "dialkylamino in which alkyl is non-tertiary $C_1$ to $C_4$ alkyl" denotes saturated, acyclic groups which may be straight or branched as exemplified by dimethylamino, diethylamino, ethylmethylamino, dipropylamino, dibutylamino, isobutylmethylamino and the like.

As used herein the terms "$C_1$ to $C_4$ alkyl", "non-tertiary $C_1$ to $C_{12}$ alkyl", and "non-tertiary $C_1$ to $C_{16}$ alkyl" denote saturated monovalent straight or branched aliphatic hydrocarbon radicals including methyl, ethyl, propyl, isopropyl, butyl, isobutyl, amyl, 1-methylbutyl, 3-methylbutyl, hexyl, isohexyl, heptyl, isoheptyl, octyl, isooctyl, 2-ethylhexyl, nonyl, 3-ethylheptyl, decyl, undecyl, dodecyl, tridecyl, 1,3,5-trimethylhexyl, 1,5-dimethyl-4-ethylhexyl, 5-methyl-2-butylhexyl, 2-propylnonyl, 2-butyloctyl, 2-pentanonyl, and the like.

The term "non-tertiary $C_1$ to $C_4$ alkoxy" includes saturated acyclic, straight or branched-chained groups such as methoxy, ethoxy, propoxy, isopropoxy, butoxy, sec-butoxy, and isobutoxy.

The best mode contemplated by the inventors of carrying out this invention will now be described so as to enable any person skilled in the art to which it pertains to make and use the same.

In view of the process aspects described hereinabove, it is readily apparent that the hydrazine compounds can react with the fluorans at one or both of the hydrazine nitrogens to obtain a five membered or a six membered ring system of Formulas I and V, respectively, or in the instances where the fluorans contains hydroxy moieties, a five membered ring compound of Formula IV or an open ring compound of Formula XII. Ordinarily, the mode in which the reaction is carried out, mixtures of the compounds of Formulas I and V and mixtures of Formulas IV and XII are

obtained. These individual compounds can be isolated if desired by conventional means, such as fractional recrystallization, preparative chromatography, or other methods commonly used in the art. However, since both of the compounds obtained by the process aspects are operative in the electrochromic recording systems, it is unnecessary to separate the compounds, and in fact, it is economically advantageous not to separate the compounds and to use them as a mixture.

In accordance with one of the aforementioned process aspects of this invention, the compounds of Formulas I and V are obtained by reacting one molecular proportion of a fluoran compound of Formula X with at least one molecular proportion of a $R^6$-substituted hydrazine of Formula XI. This reaction is conveniently carried out in an appropriate inert organic liquid, for example, a glycol such as ethylene glycol or propylene glycol; a glycol ether such as ethoxyethanol commonly known as the ethyl ether of ethylene glycol or an aliphatic amide such as N,N-dimethylformamide. Alternatively, the reaction can be run neat with an excess of the hydrazine, for example, phenylhydrazine serving as the reaction medium. This reaction is conveniently carried out at a temperature in the range of 90° to 180°C, usually at the reflux temperature for periods of approximately thirty minutes to approximately forty-eight hours. Mixtures of compounds of Formulas I and V thus obtained are isolated directly from the reaction medium after chilling. Alternatively, the desired product can be isolated by pouring the reaction mixture into a mixture of ice and water and filtering the precipitated product. The isolated product can be purified by conventional means such as recrystallization or reslurrying with a suitable organic liquid and then collected by filtration. Alternatively, the purification can be combined with the isolation by extracting the product from the mixture of ice, water and product with a suitable water immiscible organic liquid, for example, toluene and then concentrating the organic liquid solution of the product by conventional means such as evaporation or distillation. In some instances, as described in greater detail in the examples hereinbelow, the compounds of Formula V are, to a large extent, insoluble in the reaction medium and can be removed directly by filtration. The filtrate obtained is then poured into a mixture of ice and water, and a mixture comprised predominantly of a compound of Formula I and, to a smaller extent, a compound of Formula V, is isolated by filtration. The isolated mixtures can be further purified and/or separated into the individual components by conventional means such as recrystallization, reslurrying in a suitable organic liquid followed by filtration, preparative chromatography or any one of numerous other methods of purification and separation available in the art. However, since both of the compounds have activity as electrochromic color-forming materials ordinarily the mixtures obtained are used without further separation into the individual components.

In accordance with the second of the aforementioned process aspects of this invention, the compounds of Formulas IV and XII are obtained by reacting fluoran compounds of Formula XIII with a $R^6$-substituted hydrazine of Formula XI. This reaction is conveniently carried out in an appropriate inert organic liquid, for example, a glycol such as ethylene glycol or propylene glycol; or a glycol ether such as ethoxyethanol commonly known as the ethyl ether of ethylene glycol. The reaction is conveniently carried out at a temperature in the range of 90° to 180°C, usually at the reflux temperature, for periods of approximately thirty minutes to approximately forty-eight hours. Mixtures of compounds of Formulas IV and XII thus obtained are isolated by pouring the reaction mixture into a mixture of ice and water and filtering the resulting product. The isolated product can be purified by conventional means such as recrystallization or reslurrying with a suitable organic liquid followed by filtration. Alternatively, the purification can be combined with the isolation by extracting the mixture of ice, water and product with a suitable water immiscible organic liquid, for example, toluene and then concentrating the organic liquid solution of the product by conventional means such as evaporation or distillation. In some instances as described in greater detail in the examples hereinbelow, the compounds of Formula IV are, to a large extent, insoluble in the reaction medium and can be removed directly by filtration. The filtrate obtained is then poured into a mixture of ice and water, and a mixture

comprised predominantly of the compounds of Formula XII and, to a small extent, the compounds of Formula IV, is isolated by filtration. The isolated mixtures can be further purified and/or separated into the individual components by conventional means such as recrystallization, reslurrying in a suitable organic liquid followed by filtration, preparative chromatography or any one of numerous other methods of purification and separation available in the art. However, since both of the compounds have activity as electrochromic color-forming materials ordinarily the mixtures obtained are used without further separation into the individual components.

Some of the starting fluorans of Formulas X and XIII are mixtures of 5-Y and 6-Y isomers by virtue of their method of preparation as described in the patents cited hereinbelow. It will of course be appreciated that the reaction of such mixtures with hydrazines as described hereinabove results in the production of mixtures of 6-Y- and 7-Y-2,3-dihydro-spiro-[phthalazine-1(4H),9'-[9H]-xanthene]-4-ones, 5-Y- and 6-Y-spiro[1H-isoindole-1,9'-[9H]-xanthene]-3(2H)-ones and esters thereof and 9-[2-(N-$R^6$-hydrazinocarbonyl)-5-Y- and 6-Y-phenyl]-3H-xanthene-3-ones. Throughout this application, the nomenclature 5/6 and 6/7 is adopted to indicate location of the Y substituent. It is unnecessary to separate the individual isomers either in the starting fluorans or the final products because both isomers of the latter are useful in the practice of this invention.

The compounds of Formulas X and XIII hereinabove, which are required as starting materials in the preparation of the final products of Formulas I, V and XII, are known generically as fluorans. The following are examples, without limitation thereto, illustrative of a few fluorans which can be utilized in carrying out this invention: 2-anilino-6-diethylaminofluoran and 2-anilino-3-methyl-6-diethylaminofluoran and similar compounds described in U.S. Patent 3,681,390, which issued August 1, 1972; 2-dibenzyl-6-diethylaminofluoran and similar compounds described in U.S. Patent 3,839,361, which issued October 1, 1974; 2-(2-chlorophenylamino)-6-diethylaminofluoran and similar compounds described in U.S. Patent 3,920,510, which issued November 18, 1975; 2-(2-chlorophenylamino)-6-dibutylaminofluoran and similar compounds described in Japanese Patent Publication 56/162,690, which published December 14, 1981; 2-anilino-3-methyl-6-(N-methyl-N-cyclohexyl)fluoran and similar compounds described in U.S. Patent 3,959,571, which issued May 25, 1976; 2-anilino-6-[N-ethyl-N-(4-methylphenyl)amino]-fluoran and similar compounds disclosed in U.S. Patent 4,168,845, which issued September 25, 1975; 2-(2,4-dimethylphenylamino)-3-methyl-6-diethylaminofluoran disclosed in U.S. Patent 4,330,473, which issued May 18, 1982; 2-anilino-3-methyl-6-diethylamino-5'/6'-ethoxycarbonylfluoran and similar compounds disclosed in U.S. Patent 4,274,660, which issued June 23, 1981; and 2-[2-(2-pyridyl)ethyl]-6-diethylaminofluoran and similar compounds which are disclosed in Hung and Ehlinger U.S. Patent Application Serial No. 445,527, filed November 30, 1982.

The hydrazines and hydrazides of Formula XI constitute a well-known class of compounds many of which are commercially-available or are readily obtained by conventional syntheses well-known in the art. The following list exemplifies hydrazines useful in carrying out the processes of this invention. Hydrazine, 2-aminophenylhydrazine, benzylhydrazine, 2-bromophenylhydrazine, 3-bromophenylhydrazine, 4-bromophenylhydrazine, 2-chlorophenylhydrazine, 3-chlorophenylhydrazine, 4-chlorophenylhydrazine, 2,4-dichlorophenylhydrazine, 2,5-dichlorophenylhydrazine, 2,6-dichlorophenylhydrazine, 3,4-dichlorophenylhydrazine, 3,5-dichlorophenylhydrazine, 2,4-dinitrophenylhydrazine, 4-methoxyphenylhydrazine, methylhydrazine, 2-nitrophenylhydrazine, 3-nitrophenylhydrazine, 4-nitrophenylhydrazine, phenylhydrazine, 2-methylphenylhydrazine, 3-methylphenylhydrazine, 4-methylphenylhydrazine, methylhydrazinocarboxylate, acethydrazide, carbohydrazide, and oxalyldihydrazide.

The molecular structures of the compounds of this invention were assigned on the basis of the modes of synthesis and study of their infrared and nuclear magnetic resonance spectra.

The following examples will further illustrate the invention without, however, limiting it thereto. All melting points are uncorrected.

Example 1

A. With stirring, a mixture of 1250.0 ml of ethylene glycol dimethyl ether, 202.3 g of 2-anilino-3-methyl-6-diethylaminofluoran and 240.0 ml of 85 percent hydrazine hydrate was maintained at reflux over the weekend (approximately 56 hours). The resulting mixture was cooled to approximately 2°C and the solid was collected by filtration and the filtrate retained. The solid was washed with a small quantity of cold ethyl alcohol and dried to obtain 61.0 g of a mixture consisting of approximately 95.6 percent of 2'-anilino-3'-methyl-6'-diethylamino-2,3-dihydrospiro[phthalazine-1(4H),9'-[9H]-xanthene]-4-one (Formula VII: $R^1=CH_3$; $R^6=R^8=Y=H$; $R^7=C_6H_5$; $R^9=R^{10}=C_2H_5$) and approximately 4.4 percent 2-amino-2'-anilino-3'-methyl-6'-diethylamino-spiro[1H-isoindole-1,9'-[9H]-xanthene]-3(2H)-one (Formula III: $R^1=CH_3$; $R^6=R^7=Y=H$; $R^8=C_6H_5$; $R^9=R^{10}=C_2H_5$;), a yellow powder which melted at 212° to 216°C with decomposition. Significant infrared maxima appeared at 3180 $cm^{-1}$ (N-H;w) and 1680 $cm^{-1}$ (C=O;s). The nuclear magnetic resonance spectrum was consistent with the assigned structures.

B. The filtrate retained from Part A above was added slowly with stirring to 2.0 liters of cold tap water and ice was added gradually. The resulting mixture was stirred for approximately ninety minutes and the separated solid was collected by filtration. The filter cake was reslurried in 500.0 ml of isopropyl alcohol and the solids were collected by filtration. The filter cake was washed with approximately 150.0 ml of isopropyl alcohol and dried to obtain as a yellow-colored solid which melted at 229° to 232°C with decomposition, 126.4 g of a mixture consisting of 87.9 percent of 2-amino-2'-anilino-3'-methyl-6'-diethylamino-spiro[1H-isoindole-1,9'-[9H]-xanthene]-3(2H)-one (Formula III: $R^1=CH_3$; $R^6=R^7=Y=H$; $R^8=C_6H_5$; $R^9=R^{10}=C_2H_5$) and 12.1 percent 2'-anilino-3'-methyl-6'-diethylamino-2,3-dihydro-spiro[phthalazine-1(4H),9'-[9H]-xanthene]-4-one (Formula VII: $R^1=CH_3$; $R^6=R^7=Y=H$; $R^8=C_6H_5$; $R^9=R^{10}=C_2H_5$). Significant infrared maxima appeared at 3325 $cm^{-1}$ (N-H;w) and 1680 $cm^{-1}$ (C=O;s). The nuclear magnetic resonance spectrum was consistent with the assigned structures. Papers treated with an ink formulation of both products, A and B above, produced gray-colored images when traced with an applied voltage stylus.

Example 2

A stirred mixture of 40.0 ml of 2-ethoxyethanol 5.4 g of 2-anilino-3-methyl-6-[N-ethyl-N-(4-methylphenyl)]-aminofluoran and 5.0 ml of 85 percent hydrazine hydrate was maintained at approximately 100°C for approximately two hours. The reaction mixture was cooled to ambient temperature and slowly added to approximately 500.0 ml of cold tap water. The separated solid was collected by filtration, washed twice each time with 200.0 ml of tap water and dried to obtain as a white-colored solid which melted at 185° to 190°C with decomposition, 5.2 g of a mixture of

2-amino-2'-anilino-3'-methyl-6'-[N-ethyl-N-(4-methylphenyl)]amino-spiro[1H-isoindole-1,9'-[9H]-xanthene]-3(2H)-one (Formula III: $R^1=CH_3$; $R^6=R^7=Y=H$; $R^8=C_6H_5$; $R^9=4-CH_3C_6H_4$; $R^{10}=C_2H_5$) and 2'-anilino-3'-methyl-6'-[N-ethyl-N-(4-methylphenyl)]-amino-2,3-dihydro-spiro[phthalazine-1(4H),9'-[9H]-xanthene]-4-one (Formula VII: $R^1=CH_3$; $R^6=R^7=Y=H$; $R^8=C_6H_5$; $R^9=4-CH_3C_6H_4$; $R^{10}=C_2H_5$). Significant infrared maxima appeared at 3320 $cm^{-1}$ (N-H;m) and 1695 $cm^{-1}$ (C=O;s). The nuclear magnetic resonance spectrum was in accord with the assigned structure. Paper treated with an ink formulation of the product produced a gray-colored image when traced with an applied voltage stylus.

<u>Example 3</u>

A mixture of 12.2 g of 2-anilino-3-methyl-6-diethylaminofluoran and 10.8 g of phenylhydrazine was maintained at a temperature in the range of 200° to 225°C with stirring for approximately seventy-five minutes. After cooling below 100°C, the reaction mixture was poured slowly into water with stirring. The supernatant water was decanted and replaced with cold water. The solid coalesced into a single mass which was ground up in a mortar with a pestle and washed several times with cold water. After drying there was obtained as an orange-colored solid which melted over the range of 80° to 108°C, 10.6 g of a mixture of 2-phenylamino-2'-anilino-3'-methyl-6'-diethylamino-spiro-[1H-isoindole-1,9'-[9H]-xanthene]-3(2H)-one (Formula III:

$R^1=CH_3$; $R^6=R^8=C_6H_5$; $R^7=Y=H$; $R^9=R^{10}=C_2H_5$) and 3-phenyl-2'-anilino-3'-methyl-6'-diethylamino-2,3-dihydro-spiro[phthalazine-1(4H),9'-[9H]-xanthene]-4-one (Formula VII: $R^1=CH_3$; $R^6=R^8=C_6H_5$; $R^7=Y=H$; $R^9=R^{10}=C_2H_5$). Significant infrared maxima appeared at 2975 $cm^{-1}$ (N-H;w) and 1705 $cm^{-1}$ (C=O;s). The nuclear magnetic resonance spectrum was concordant with the assigned structures. Paper treated with an ink formulation of the product produced a gray-colored image when traced with an applied voltage stylus.

Proceeding in a manner similar to that described in Example 1 above, the appropriate substituted fluoran described in the second column of Part 1 of Table A hereinbelow was interacted with hydrazine hydrate or other substituted hydrazine described in the third column of Part 1 in the organic medium given in the fourth column of Part 1 at a temperature of the mixture given in the fifth column of Part 1 for the period of time indicated in the sixth column of Part 1. The product obtained is given in the seventh column of Part 2 of Table A having the structural formula referred to in the third column of Part 2 with its physical appearance described in the fourth column of Part 2. Its melting point is shown in the fifth column of Part 2, significant infrared maxima are indicated in the sixth column of Part 2, and nuclear magnetic resonance spectral analysis are shown in the seventh column of Part 2. The color produced when a paper sheet treated with an ink

formulation containing the product was traced with an applied voltage stylus is described in the eighth column of Part 2.

TABLE A - Part 1

| Example No. | Starting Fluoran | Hydrazine Hydrate | Organic Medium | Temper-ature | Reaction Time |
|---|---|---|---|---|---|
| 4 | 25.0 g 2-(2,4-Dimethylphenyl)amino-3-methyl-6-diethylaminofluoran | 25.0 ml of 85 percent | 200.0 ml of 2-Ethoxy-ethanol | Reflux | 1 hour |
| 5 | 81.0 g of Rhodamine B | 50.0 ml of 85 percent | 500.0 ml of 2-Ethoxy-ethanol | Reflux | 1 hour |
| 6 | 7.0 g 2-Benzylamino-3-methyl-6-diethylaminofluoran | 10.0 ml of 85 percent | 80.0 ml of 2-Ethoxy-ethanol | Reflux | 2 hours |
| 7 | 3.4 g 2-Amino-3-methyl-6-diethyl-aminofluoran | 6.0 ml of 85 percent | 50.0 ml of 2-Ethoxy-ethanol | Reflux | 2 hours |
| 8 | 212.0 g 2-Amino-6-diethylamino-fluoran | 250.0 ml of 85 percent | 1250.0 ml of 2-Ethoxy-ethanol | Reflux | 1 hour |

TABLE A - Part 1 (cont.)

| Example No. | Starting Fluoran | Hydrazine Hydrate | Organic Medium | Temperature | Reaction Time |
|---|---|---|---|---|---|
| 9 | 5.3 g 2-[N-Acetyl-N-2-(2-pyridyl)-ethyl]amino-6-diethylaminofluoran | 5.0 ml of 85 percent | 40.0 ml of 2-Ethoxy-ethanol | 80° to 82°C | 1 hour |
| 10 | 3.0 g 2-[2-(2-Pyridyl)ethyl]amino-3-methyl-6-diethylaminofluoran | 3.0 ml of 85 percent | 15.0 ml of N,N-Dimethyl-formamide | 40°C | 19 hours |
| 11 | 3.0 g 2-[2-(2-Pyridyl)ethyl]amino-6-diethylaminofluoran | 3.0 ml of 85 percent | 15.0 ml of N,N-Dimethyl-formamide | 40°C | 19 hours |
| 12 | 11.0 g 2-Anilino-3-methyl-6-diethylamino-5'/6'-ethoxycarbonyl-fluoran | 6.0 ml of 85 percent | 30.0 ml of 2-Ethoxy-ethanol | 100°C | 1 hour |
| 13 | 14.0 g 2-Dibenzylamino-6-diethyl-amino-5'/6'-phenylmethoxycarbonyl-fluoran | 6.0 ml of 85 percent | 30.0 ml of 2-Ethoxy-ethanol | 100°C | 1 hour |
| 14 | 3.2 g 2-Amino-6-diethylamino-5'/6'-carboxyfluoran | 8.0 ml of 85 percent | 50.0 ml of 2-Ethoxy-ethanol | Reflux | 2 hours |

TABLE A - Part 1 (cont.)

| Example No. | Starting Fluoran | Hydrazine Hydrate | Organic Medium | Temperature | Reaction Time |
|---|---|---|---|---|---|
| 15 | 10.0 g 2-(2-Chlorophenyl)amino-6-diethylaminofluoran | 10.0 ml of 85 percent | 50.0 ml of 2-Ethoxyethanol | 100°C | 30 minutes |
| 16 | 8.8 g 2-(2-Chlorophenyl)amino-6-dibutylaminofluoran | 4.75 ml of 85 percent | 50.0 ml of 2-Ethoxyethanol | 100°C | 2 hours |
| 17 | 3.0 g 2-Pyrrolyl-6-diethylaminofluoran | 4.0 ml of 85 percent | 20.0 ml of 2-Ethoxyethanol | 110°C | 1 hour |
| 18 | 23.0 g 2-Dibenzylamino-6-diethylaminofluoran | 23.0 ml of 85 percent | 250.0 ml of 2-Ethoxyethanol | 110°C | 45 minutes |
| 19 | 3.0 g 2-Octylamino-6-diethylaminofluoran | 4.0 ml of 85 percent | 20.0 ml of 2-Ethoxyethanol | 110°C | 1 hour |
| 20 | 19.3 g 2-Methyl-6-diethylaminofluoran | 23.0 ml of 85 percent | 100.0 ml of 2-Ethoxyethanol | 110°C | 1 hour |

TABLE A - Part 1 (cont.)

| Example No. | Starting Fluoran | Hydrazine Hydrate | Organic Medium | Temperature | Reaction Time |
|---|---|---|---|---|---|
| 21 | 18.6 g 2-Bromo-3-methyl-6-diethylaminofluoran | 12.0 ml of 85 percent | 100.0 ml of 2-Ethoxyethanol | 100°C | 1 hour |
| 22 | 19.0 g 2-Anilino-3-methyl-6-pyrrolidylfluoran | 23.0 ml of 85 percent | 100.0 ml of 2-Ethoxyethanol | 110°C | 30 minutes |
| 23 | 20.7 g 2-Anilino-3-methyl-6-(N-methyl-N-cyclohexylamino)fluoran | 23.0 ml of 85 percent | 100.0 ml of 2-Ethoxyethanol | 110°C | 30 minutes |
| 24 | 5.0 g 2-Anilino-6-diethylaminofluoran | 5.0 ml of 85 percent | 60.0 ml of 2-Ethoxyethanol | 110°C | 30 minutes |
| 25 | 14.3 g 2-Anilino-3-methyl-6-diethylaminofluoran | 9.0 g of Carbohydrazide | (none) | 180°C | 10 minutes |

TABLE A - Part 1 (cont.)

| Example No. | Starting Fluoran | Hydrazine Hydrate | Organic Medium | Temperature | Reaction Time |
|---|---|---|---|---|---|
| 26 | 12.2 g 2-Anilino-3-methyl-6-diethylaminofluoran | 11.8 g of Oxalyldihydrazide | 50.0 ml of Ethylene glycol | Reflux | 2 hours |
| 27 | 14.3 g 2-Anilino-3-methyl-6-diethylaminofluoran | 14.8 g of Acetylhydrazide | (none) | 160°C | 1 hour |

TABLE A - Part 2

| Example No. | Product | Product Formula | Physical Appearance | Melting Point | Significant Infrared | NMR | Produced Image Colc |
|---|---|---|---|---|---|---|---|
| 4 | 25.6 g of a Mixture of 2-amino-2'-(2,4-dimethylphenyl)amino-3'-methyl-6'-diethylamino-spiro[1H-isoindole-1,9'-[9H]-xanthene]-3(2H)-one and 2'-(2,4-dimethylphenyl)amino-3'-methyl-6'-diethylamino-2,3-dihydro-spiro-[phthalazine-1(4H),9'-[8H]-xanthene]-4-one | III & VII: $R^1=CH_3$; $R^6=R^7=Y=H$; $R^8=2,4-(CH_3)_2-C_6H_3$; $R^9=R^{10}=C_2H_5$ | White powder | 114° to 132°C | 3460 $cm^{-1}$ to 3180 $cm^{-1}$ (N-H;b) 1695 $cm^{-1}$ (C=O;s) | Consistent | Gray |
| 5 | 52.1 g of a Mixture of 2-Amino-3',6'-bis(diethylamino)-spiro[1H-isoindole-1,9'-[9H]-xanthene]-3(2H)-one and 3',6'-bis(diethylamino)-2,3-dihydro-spiro[phthalazine-1(4H),9'-[9H]-xanthene]-4-one | II & VI: $R=R^6=Y=H$; $R^1=(C_2H_5)_2N$; $R^9=R^{10}=C_2H_5$ | White powder | 109° to 112°C | 3337 $cm^{-1}$ (N-H;w) 1700 $cm^{-1}$ (C=O;s) | Consistent | Red-purple |

TABLE A - Part 2 (cont.)

| Example No. | Product | Product Formula | Physical Appearance | Melting Point | Significant Infrared | NMR | Produced Image Color |
|---|---|---|---|---|---|---|---|
| 6 | 6.8 g of a Mixture of 2-amino-2'-benzylamino-3'-methyl-6'-diethyl-amino-spiro[1H-isoindole-1,9'-[9H]-xanthene]-3(2H)-one and 2'-benzylamino-3'-methyl-6'-diethylamino-2,3-dihydro-spiro-[phthalazine-1(4H),9'-xanthene]-4-one | III & VII: $R^1=CH_3$; $R^6=R^7=Y=H$; $R^8=C_6H_5CH_2$; $R^9=R^{10}=C_2H_5$ | White powder | 114° to 117°C | 3400 $cm^{-1}$ (N-H;b) 1690 $cm^{-1}$ (C=O;s) | Consistent | Black |
| 7 | 3.62 g 2-Amino-2'-amino-3'-methyl-6'-diethylamino-spiro[1H-isoindole-1,9'-[9H]-xanthene]-3(2H)-one | III: $R^1=CH_3$; $R^6=R^7=R^8=Y=H$; $R^9=R^{10}=C_2H_5$ | Tan powder | 137° to 141°C | 3380 $cm^{-1}$ (N-H;s) 1695 $cm^{-1}$ (C=O;s) | Consistent | Gray |

TABLE A - Part 2 (cont.)

| Example No. | Product | Product Formula | Physical Appearance | Melting Point | Significant Infrared | NMR | Produced Image Color |
|---|---|---|---|---|---|---|---|
| 8 | 220.4 g of a Mixture of 2,2'-(diamino)-6'-diethylamino-spiro[1H-isoindole-1,9'-[9H]-xanthene]-3(2H)-one and 2'-amino-6'-diethylamino-2,3-dihydro-spiro-[phthalazine-1(4H),9'-[9H]-xanthene]-4-one | III & VII: $R^1=R^6=R^7=R^8=Y=H$; $R^9=R^{10}=C_2H_5$ | Light gray solid | 168° to 171°C | 3320 $cm^{-1}$ (N-H;m) 1690 $cm^{-1}$ (C=O;s) | Consistent | Red-gray |
| 9 | 4.4 g of a Mixture of 2-amino-2'-[N-acetyl-N-2-(2-pyridyl)ethyl]-amino-6'-diethylamino-spiro[1H-isoindole-1,9'-[9H]-xanthene]-3(2H)-one and 2'-[N-acetyl-N-2-(2-pyridyl)-ethyl]amino-6-diethylamino-2,3-dihydro-spiro[phthalazine-1(4H),9'-[9H]-xanthene]-4-one | III & VII: $R^1=R^6=Y=H$; $R^9=R^{10}=C_2H_5$; $R^8=C_2H_4C_5H_4N$; $R^7=CH_3CO$ | Pale green-brown powder | 104° to 108°C | 3100 $cm^{-1}$ to 3500 $cm^{-1}$ (N-H;b) 1635 $cm^{-1}$ (C=O;s) | Consistent | Gray |

TABLE A - Part 2 (cont.)

| Example No. | Product | Product Formula | Physical Appearance | Melting Point | Significant Infrared | NMR | Produced Image Color |
|---|---|---|---|---|---|---|---|
| 10 | 2.3 g of a Mixture of 2-amino-2'-[2-(2-pyridyl)ethyl]amino-3-methyl-6'-diethylamino-spiro[1H-isoindole-1,9'-[9H]-xanthene]-3(2H)-one and 2'-[2-(2-pyridyl)ethyl]amino-3-methyl-6'-diethylamino-2,3-dihydro-spiro[phthalazine-1(4H),9'-[9H]-xanthene]-4-one | III & VII: $R^1=CH_3$; $R^6=R^7=Y=H$; $R^8=C_2H_4C_5H_4N$; $R^9=R^{10}=C_2H_5$ | White powder | 100° to 105°C | 3000 $cm^{-1}$ to 2850 $cm^{-1}$ (N-H;b) 1670 $cm^{-1}$ (C=O;s) | Consistent | Black |
| 11 | 2.7 g of a Mixture of 2-amino-2'-[2-(2-pyridyl)ethyl]amino-6'-diethylamino-spiro[1H-isoindole-1,9'-[9H]-xanthene]-3(2H)-one and 2'-[2-(2-pyridyl)-ethyl]amino-6'-diethylamino-2,3-dihydro-spiro[phthalazine-1(4H),9'-[9H]xanthene]-4-one | III & VII: $R^1=R^6=R^7=Y=H$; $R^8=C_2H_4C_5H_4N$; $R^8=R^{10}=C_2H_5$ | Beige powder | 100° to 105°C | 3000 $cm^{-1}$ to 2850 $cm^{-1}$ (N-H;b) 1670 $cm^{-1}$ (C=O;s) | Consistent | Black |

TABLE A - Part 2 (cont.)

| Example No. | Product | Product Formula | Physical Appearance | Melting Point | Significant Infrared | NMR | Produced Image Color |
|---|---|---|---|---|---|---|---|
| 12 | 8.8 g of a Mixture of 2-amino-2'-anilino-3'-methyl-6'-diethylamino-5/6-ethoxycarbonyl-spiro[1H-isoindole-1,9'-[9H]-xanthene]-3(2H)-one and 2'-anilino-3'-methyl-6'-diethylamino-5/6-ethoxycarbonyl-2,3-dihydro-spiro[phthalazine-1(4H),-9'-[9H]-xanthene-4-one | III & VII: $R^1=CH_3$; $R^6=C_2H_5OCO$; $R^7=H$; $R^8=C_6H_5$; $R^9=R^{10}=C_2H_5$ | Brown powder | 107°C Decomposed | 3415 $cm^{-1}$ to 3140 $cm^{-1}$ (N-H;b) 1675 $cm^{-1}$ (C=O;s) | Consistent | Blue-black |
| 13 | 8.3 g of a Mixture of 2-amino-2'-dibenzylamino-6'-diethylamino-5/6-phenylmethoxycarbonyl-spiro[1H-isoindole-1,9'-[9H]-xanthene]-3(2H)-one and 2'-dibenzylamino-6'-diethylamino-5/6-phenylmethoxy-2,3-dihydro-spiro[phthalazine-1(4H),9'-[9H]-xanthene]-4-one | III & VII: $R^1=H$; $R^6=C_6H_5CH_2OCO$; $R^7=R^8=C_6H_5-CH_2$; $R^9=R^{10}=C_2H_5$; | Pale brown powder | 90°C Decomposed | 3480 $cm^{-1}$ to 3360 $cm^{-1}$ (N-H;b) 1685 $cm^{-1}$ (C=O;m) | Consistent | Blue-green |

TABLE A - Part 2 (cont.)

| Example No. | Product | Product Formula | Physical Appearance | Melting Point | Significant Infrared | NMR | Produced Image Color |
|---|---|---|---|---|---|---|---|
| 14 | 1.05 g of a Mixture of 2,2'-(diamino)-6'-diethylamino-5/6-carboxy-spiro[1H-isoindole-1,9'-[9H]-xanthene]-3(2H)-one and 2'-amino-6'-dimethylamino-5/6-carboxy-2,3-dihydro-spiro[phthalazine-1(4H),9'-[9H]-xanthene]-4-one | III & VII: $R^1=R^7=H$; $R^8=NH_2$; $R^9=R^{10}=C_2H_5$; $R^6=COOH$ | Tan solid | 242°C Decomposed | 3450 $cm^{-1}$ to 3200 $cm^{-1}$ (N-H;w) 1695 $cm^{-1}$ (C=O;s) | Consistent | Gray |
| 15 | 8.6 g of a Mixture of 2-amino-2'-(2-chlorophenyl)amino-6'-diethylamino-spiro[1H-isoindole-1,9'-[9H]-xanthene-3(2H)-one and 2'-(2-chlorophenyl)amino-6'-diethylamino-2,3-dihydro-spiro[phthalazine-1(4H),9'-[9H]-xanthene]-4-one | III & VII: $R^1=R^6=R^7=Y=H$; $R^8=2-ClC_6H_4$; $R^9=R^{10}=C_2H_5$ | Pale tan powder | 175°C Decomposed | 3415 $cm^{-1}$ (N-H;w) 1687 $cm^{-1}$ (C=O;s) | Consistent | Black |

TABLE A - Part 2 (cont.)

| Example No. | Product | Product Formula | Physical Appearance | Melting Point | Significant Infrared | NMR | Produced Image Color |
|---|---|---|---|---|---|---|---|
| 16 | 2.2 g of a Mixture of 2-amino-2'-(2-chlorophenyl)amino-6'-dibutylamino-spiro[1H-isoindole-1,9'-[9H]-xanthene]-3(2H)-one and 2'-(2-chlorophenyl)-amino-6'-dibutylamino-2,3-dihydro-spiro-[phthalazine-1(4H),9'-[9H]-xanthene]-4-one | III & VII: $R^1=R^6=R^7=Y=H$; $R^8=2-ClC_6H_4$; $R^9=R^{10}=C_4H_9$ | White powder | 184° to 185°C | 3410 $cm^{-1}$ (N-H;w) 1690 $cm^{-1}$ (C=O;m) | Consistent | Black |
| 17 | 2.7 g of a Mixture of 2-amino-2'-pyrrolyl-6'-diethylamino-spiro-[1H-isoindole-1,9'-[9H]-xanthene]-3(2H)-one and 2'-pyrrolyl-6'-diethylamino-2,3-dihydro-spiro[phthalazine-1(4H),9'-[9H]-xanthene]-4-one | III & VII: $R^1=R^6=Y=H$; $R^7=R^8=C_4H_8$; $R^9=R^{10}=C_2H_5$ | Pale gray powder | 163°C Decomposed | 3410 $cm^{-1}$ (N-H;w) 1692 $cm^{-1}$ (C=O;s) | Consistent | Grape |

TABLE A - Part 2 (cont.)

| Example No. | Product | Product Formula | Physical Appearance | Melting Point | Significant Infrared | NMR | Produced Image Color |
|---|---|---|---|---|---|---|---|
| 18 | 24.2 g of a Mixture of 2-amino-2'-dibenzylamino-6'-diethylamino-spiro[1H-isoindole-1,9'-[9H]-xanthene]-3(2H)-one and 2'-dibenzylamino-6'-diethylamino-2,3-dihydro-spiro[phthalazine-1(4H),9'-[9H]-xanthene]-4-one | III & VII: $R^1=R^6=Y=H$; $R^7=R^8=CH_2C_6H_5$; $R^9=R^{10}=C_2H_5$ | Dark pink powder | 85° to 140°C | 3680 $cm^{-1}$ (N-H;w) 1625 $cm^{-1}$ (C=O;m) | Consistent | Green |
| 19 | 3.5 g of a Mixture of 2-Amino-2'-octylamino-6'-diethylamino-spiro-[1H-isoindole-1,9'-[9H]-xanthene]-3(2H)-one and 2'-octylamino-6'-diethylamino-2,3-dihydro-spiro[phthalazine-1(4H),9'-[9H]-xanthene]-4-one | III & VII: $R^1=R^6=R^7=Y=H$; $R^8=C_8H_{17}$; $R^9=R^{10}=C_2H_5$ | Dark pink powder | 123° to 140°C | 3410 $cm^{-1}$ (N-H;m) 1680 $cm^{-1}$ (C=O;s) | Consistent | Green-gray |

TABLE A - Part 2 (cont.)

| Example No. | Product | Product Formula | Physical Appearance | Melting Point | Significant Infrared | NMR | Produced Image Color |
|---|---|---|---|---|---|---|---|
| 20 | 17.9 g of a Mixture of 2-amino-2'-methyl-6'-diethylamino-spiro[1H-isoindole-1,9'-[9H]-xanthene]-3(2H)-one and 2'-methyl-6'-diethylamino-2,3-dihydro-spiro-[phthalazine-1(4H),9'-[9H]-xanthene]-4-one | II & VI: $R^1=R^6=Y=H$; $R=CH_3$; $R^9=R^{10}=C_2H_5$ | Pink powder | 153°C Decomposed | 3410 $cm^{-1}$ (N-H;w) 1695 $cm^{-1}$ (C=O;s) | Consistent | Rose |
| 21 | 10.6 g of a Mixture of 2-amino-2'-bromo-3'-methyl-6'-diethylamino-spiro[1H-isoindole-1,9'-[9H]-xanthene]-3(2H)-one and 2'-bromo-3'-methyl-6'-diethylamino-2,3-dihydro-spiro-[phthalazine-1(4H),9'-[9H]-xanthene]-4-one | II & VI: R=Br; $R^1=R^6=Y=H$; $R^9=R^{10}=C_2H_5$ | White powder | 225°C | 3600 $cm^{-1}$ to 3160 $cm^{-1}$ (N-H;b) 1700 $cm^{-1}$ (C=O;s) | Consistent | Red |

TABLE A - Part 2 (cont.)

| Example No. | Product | Product Formula | Physical Appearance | Melting Point | Significant Infrared | NMR | Produced Image Color |
|---|---|---|---|---|---|---|---|
| 22 | 19.7 g of a Mixture of 2-amino-2'-anilino-3'-methyl-6'-pyrrolidyl-spiro[1H-isoindole-1,9'-[9H]-xanthene]-3(2H)-one and 2'-anilino-3'-methyl-6'-pyrrolidyl-2,3-dihydro-spiro[phthalazine-1(4H),9'-[9H]-xanthene]-4-one | III & VII: $R^1=CH_3$; $R^6=R^7=Y=H$; $R^8=C_6H_5$; $R^9=R^{10}=C_4H_8$ | Beige powder | 180°C Decomposed | 3020 $cm^{-1}$ (N-H;s) 1680 $cm^{-1}$ (C=O;w) | Consistent | Black |
| 23 | 16.6 g of a Mixture of 2-amino-2'-anilino-3'-methyl-6'-(N-methyl-N-cyclohexylamino)-spiro-[1H-isoindole-1,9'-[9H]-xanthene]-3(2H)-one and 2'-anilino-3'-methyl-6'-(N-methyl-N-cyclohexylamino)-2,3-dihydro-spiro[phthalazine-1(4H),9'-[9H]-xanthene]-4-one | III & VII: $R^1=R^{10}=CH_3$; $R^6=R^7=Y=H$; $R^8=C_6H_5$; $R^9=C_6H_{11}$ | Pale pink powder | 212°C Decomposed | 3410 $cm^{-1}$ (N-H;w) 1685 $cm^{-1}$ (C=O;s) | Consistent | Black |

TABLE A - Part 2 (cont.)

| Example No. | Product | Product Formula | Physical Appearance | Melting Point | Significant Infrared | NMR | Produced Image Color |
|---|---|---|---|---|---|---|---|
| 24 | 4.2 g of a Mixture of 2-amino-2'-anilino-6'-diethylamino-spiro[1H-isoindole-1,9'-[9H]-xanthene]-3(2H)-one and 2'-anilino-6'-diethyl-amino-2,3-dihydro-spiro-[phthalazine-1(4H),9'-[9H]-xanthene]-4-one | III & VII: $R^1=R^6=R^7=Y=H$; $R^8=C_6H_5$; $R^9=R^{10}=C_2H_5$ | Pale gray powder | 150°C Decomposed | 3410 $cm^{-1}$ (N-H;w) 1685 $cm^{-1}$ (C=O;m) | Consistent | Green |
| 25 | 14.3 g 2-(Hydrazino-carbonyl)amino-2'-anilino-3'-methyl-6'-diethylamino-spiro[1H-isoindole-1,9'-[9H]-xanthene]-3(2H)-one | III: $R^1=CH_3$; $R^7=Y=H$; $R^6=NH_2NHCO$; $R^9=R^{10}=C_2H_5$ | Pink powder | 226°C Decomposed | 3500 $cm^{-1}$ to 3200 $cm^{-1}$ (N-H;b) 1700 $cm^{-1}$ (C=O;s) | Consistent | Gray |

TABLE A - Part 2 (cont.)

| Example No. | Product | Product Formula | Physical Appearance | Melting Point | Significant Infrared | NMR | Produced Image Color |
|---|---|---|---|---|---|---|---|
| 26 | 11.8 g 2-(Hydrazino-oxoacetyl)amino-2'-anilino-3'-methyl-6'-diethylamino-spiro-[1H-isoindole-1,9'-[9H]-xanthene]-3(2H)-one | III: $R^1$= $CH_3$; $R^6$= $NH_2NHCOCO$; $R^7$=Y=H; $R^8$=$C_6H_5$; $R^9$=$R^{10}$= $C_2H_5$ | Peach powder | 222°C Decomposed | 3500 $cm^{-1}$ to 3200 $cm^{-1}$ (N-H;b) 1695 $cm^{-1}$ (C=O;s) | Consistent | Gray |
| 27 | 15.3 g 2-Acetylamino-2'-anilino-3'-methyl-6'-diethylamino-spiro-[1H-isoindole-1,9'-[9H]-xanthene]-3(2H)-one | III: $R^1$= $CH_3$; $R^6$= $CH_3CO$; $R^7$= Y=H; $R^8$= $C_6H_5$; $R^9$= $R^{10}$=$C_2H_5$ | Pale gray powder | 115° to 150°C | 3420 $cm^{-1}$ (N-H;w) 1692 $cm^{-1}$ (C=O;s) | Consistent | Gray |

Example 28

A mixture of 10.0 g of 4,5-dibromo-3,6-dihydroxyfluoran, 100.0 ml of 2-ethoxyethanol and 20.0 ml of 85 percent hydrazine hydrate was refluxed with stirring for approximately eighteen hours. The resulting mixture was cooled to ambient temperature and slowly poured into 600.0 ml of water. The mixture was adjusted to pH 5.0 by gradually adding glacial acetic acid. The solid which formed was collected by filtration, washed twice, each time with 50.0 ml of water, and dried to obtain, as a white solid which melted at 191°C with decomposition, 8.7 g of a mixture consisting of approximately 67 percent of 2-amino-4',5'-dibromo-3',6'-dihydroxy-spiro[1H-isoindole-1,9'-[9H]-xanthene]-3(2H)-one (Formula IV: $R=R^5=R^6=Y=H$; $R^2=R^3=Br$) and approximately 23 percent of 4,5-dibromo-6-hydroxy-9-(2-hydrazocarbonylphenyl)-[3H]-xanthene-3-one (Formula VIII: $R=R^5=R^6=Y=H$; $R^2=R^3=Br$). A significant infrared maximum appeared at 1690 $cm^{-1}$ (C=O;s). The nuclear magnetic resonance spectrum was in accord with the assigned structure. Paper treated with an ink formulation of the product produced a red-colored image when traced with an applied voltage stylus.

Example 29

A. With stirring, a mixture of 15.0 g of 2,4,5,7-tetrabromo-3,6-dihydroxyfluoran, 20.0 ml of 85 percent hydrazine hydrate and 100.0 ml of 2-ethoxyethanol was maintained at reflux for approximately one hour. To the reaction mixture, 50.0 ml of 2-ethoxyethanol and 10.0 ml of 85 percent hydrazine hydrate were added and reflux continued overnight. In the morning, the mixture was cooled to ambient temperature and a solid was collected by filtration and washed with water and dried to obtain 0.77 g of 2-amino-2',4',5',7'-tetrabromo-3',6'-dihydroxy-spiro[1H-isoindole-1,9'-[9H]-xanthene]-3(2H)-one (Formula IV: $R=R^2=R^3=R^5=Br$; $R^6=Y=H$), a white solid which melted over the range of 233° to 255°C with decomposition. Significant infrared maxima appeared at 3220 $cm^{-1}$ (N-H;m) and 1680 $cm^{-1}$ (C=O;s). The nuclear magnetic resonance spectrum was concordant with the assigned structures.

B. The filtrate from Part A above was added slowly into 1.0 liter of water and the pH adjusted to 5.7 with the gradual addition of glacial acetic acid. The solid which formed was collected by filtration, washed twice, each time with 75.0 ml of water, and dried to obtain, a pale pink solid which melted over the range of 187° to 205°C with decomposition, 12.55 g of a mixture of approximately 77 percent of 2-amino-2',4',5',7'-tetrabromo-3',6'-dihydroxy-spiro[1H-isoindole-1,9'-[9H]-xanthene]-3(2H)-one (Formula IV: $R=R^2=R^3=R^5=Br$; $R^6=Y=H$) and 23 percent of 2,4,5,7-tetra-

bromo-6-hydroxy-9-(2-hydrazocarbonylphenyl)-[3H]-xanthene-3-one (Formula VIII: $R=R^2=R^3=R^5=Br$; $R^6=Y=H$). Significant infrared maxima appeared at 3200-3300 $cm^{-1}$ (N-H;b) and 1690 $cm^{-1}$ (C=O;s). The nuclear magnetic resonance spectrum was consistent with the assigned structures. Paper treated with an ink formulation of both products A and B above, produced pink-colored images when traced with an applied voltage stylus.

Proceeding in a manner similar to that described in Example 28 above, the appropriate substituted fluoran described in the second column of Part 1 of Table B hereinbelow was interacted with hydrazine hydrate or other substituted hydrazine described in the third column of Part 1 in the organic medium given in the fourth column of Part 1 below at a temperature of the mixture given in the fifth column of Part 1 for the period of time indicated in the sixth column of Part 1 below. The product obtained is given in the second column of Part 2 of Table B having the structural formula indicated in the third column of Part 2 with its physical appearance described in the fourth column of Part 2. Its melting point is given in the fifth column of Part 2, significant infrared maxima are indicated in the sixth column of Part 2 and its nuclear magnetic resonance spectral analysis in the seventh column of Part 2. The color produced when a paper sheet treated with an ink formulation containing the product was traced with an applied voltage stylus is given in the eighth column of Part 2.

TABLE B - Part 1

| Example No. | Starting Fluoran | Hydrazine Hydrate | Organic Medium | Temperature | Reaction Time |
|---|---|---|---|---|---|
| 30 | 6.8 g 3,6-Dihydroxy-4',5',6',7'-tetrachlorofluoran | 30.0 ml 85 percent | 200.0 ml 2-Ethoxyethanol | Reflux | 51 hours |
| 31 | 45.0 g 3,6-Dihydroxyfluoran | 80.0 ml 85 percent | 350.0 ml 2-Ethoxyethanol | Reflux | 1 day |
| 32 | 20.0 g 2,4,5,7-Tetraiodo-3,6-dihydroxyfluoran | 35.0 ml 85 percent | 300.0 ml 2-Ethoxyethanol | Reflux | 19 hours |
| 33 | 25.0 g 2,7-Dichloro-3,6-dihydroxyfluoran | 40.0 ml 85 percent | 200.0 ml 2-Ethoxyethanol | Reflux | 20 hours |
| 34 | 4.9 g 3,6-Dihydroxy-5'-amino-fluoran | 10.0 ml 85 percent | 100.0 ml 2-Ethoxyethanol | Reflux | 20 hours |
| 35 | 14.0 g 4,5-Diiodo-3,6-dihydroxy-fluoran | 25.0 ml 85 percent | 150.0 ml 2-Ethoxyethanol | Reflux | 19 hours |
| 36 | 25.0 g 5'/6'-Carboxy-3,6-dihydroxyfluoran | 40.0 ml 85 percent | 200.0 ml 2-Ethoxyethanol | Reflux | 4 hours |
| 37 | 22.6 g 2,4,5,7-Tetraiodo-3,6-dihydroxy-4',5',6',7'-tetra-chlorofluoran | 40.0 ml 85 percent | 150.0 ml 2-Ethoxyethanol | Reflux | 19 hours |

TABLE B - Part 2

| Example No. | Product | Product Formula | Physical Appearance | Melting Point | Significant Infrared | NMR | Produced Image Color |
|---|---|---|---|---|---|---|---|
| 30 | 2.0 g of a Mixture of 2-amino-3',6'-dihydroxy-4,5,6,7-tetrachloro-spiro[1H-isoindole-1,9'-[9H]-xanthene]-3(2H)-one and 6-hydroxy-9-(2-hydrazocarbonyl-3,4,5,6-tetrachlorophenyl)-[3H]-xanthene-3-one | IV & VIII: $R=R^2=R^3=R^5=R^6=H$; $Y=Cl_4$ | Orange powder | 270°C Decomposed | 3240 $cm^{-1}$ (N-H;b) 1690 $cm^{-1}$ (C=O;s) | Consistent | Orange |
| 31 | 47.7 g of a Mixture of approx. 60% 2-amino-3',6'-dihydroxy-spiro-[1H-isoindole-1,9'-[9H]-xanthene]-3(2H)-one and 40% 6-hydroxy-9-(2-hydrazocarbonylphenyl)-xanthene-3-one | IV & VIII: $R=R^2=R^3=R^5=R^6=Y=H$ | White powder | 289°C Decomposed | 3270 $cm^{-1}$ (N-H;s) 1700 $cm^{-1}$ (C=O;s) | Consistent | Red |

TABLE B - Part 2 (cont.)

| Example No. | Product | Product Formula | Physical Appearance | Melting Point | Significant Infrared | NMR | Produced Image Color |
|---|---|---|---|---|---|---|---|
| 32 | 7.2 g of a Mixture of approx. 62% 2-amino-2',4',5',7'-tetraiodo-3',6'-dihydroxy-spiro-[1H-isoindole-1,9'-[9H]-xanthene]-3(2H)-one and 38% 2,4,5,7-tetraiodo-6-hydroxy-9-(2-hydrazocarbonylphenyl)-[3H]-xanthene-3-one | IV & VIII: $R=R^2=R^3=R^5=$ I; $R^6=Y=H$ | Tan powder | 217°C Decomposed | 3180 to 3280 $cm^{-1}$ (N-H;m) 1696 $cm^{-1}$ (C=O;s) | Consistent | Red |
| 33 | 25.0 g of a Mixture of approx. 60% 2-amino-2',7'-dichloro-3',6'-dihydroxy-spiro-[1H-isoindole-1,9'-[9H]-xanthene-3(2H)-one and 40% 2,7-dichloro-6-hydroxy-9-(2-hydrazocarbonylphenyl)-[3H]-xanthene-3-one | IV & VIII: $R=R^5=Cl$; $R^2=R^3=R^6=$ $Y=H$ | Tan powder | 286° to 289°C Decomposed | 3080 to 3310 $cm^{-1}$ (N-H;b) 1710 $cm^{-1}$ (C=O;s) | Consistent | Red |

TABLE B - Part 2 (cont.)

| Example No. | Product | Product Formula | Physical Appearance | Melting Point | Significant Infrared | NMR | Produced Image Color |
|---|---|---|---|---|---|---|---|
| 34 | 5.0 g of a Mixture of approx. 83% of 2,5-diamino-3',6'-dihydroxy-spiro-[1H-isoindole-1,9'-[9H]-xanthene]-3(2H)-one and 17% 6-hydroxy-9-(2-hydrazocarbonyl-5-aminophenyl)-3H-xanthene-3-one | IV & VIII: $R=R^2=R^3=R^5=R^6=H$; $Y=NH_2$ | Orange powder | 217° to 221°C Decomposed | 3260 $cm^{-1}$ (N-H;m) 1690 $cm^{-1}$ (C=O;s) | Consistent | Orange |
| 35 | 7.8 g of a Mixture of 2-amino-4',5'-diiodo-3',6'-dihydroxy-spiro-[1H-isoindole-1,9'-[9H]-xanthene]-3(2H)-one and 4,5-diiodo-6-hydroxy-9-(2-hydrazocarbonylphenyl)-3H-xanthene-3-one | IV & VIII: $R=R^5=R^6=Y=H$; $R^2=R^3=I$ | Light yellow powder | 216° to 218°C Decomposed | 3200 $cm^{-1}$ to 3300 $cm^{-1}$ (N-H;s) 1718 $cm^{-1}$ and 1700 $cm^{-1}$ (C=O;s) | Consistent | Red |

TABLE B - Part 2 (cont.)

| Example No. | Product | Product Formula | Physical Appearance | Melting Point | Significant Infrared | NMR | Produced Image Color |
|---|---|---|---|---|---|---|---|
| 36 | 6.8 g of a Mixture of 2-amino-5/6-carboxy-3',6'-dihydroxy-spiro-[1H-isoindole-1,9'-[9H]-xanthene]-3(2H)-one and 6-hydroxy-9-(2-hydrazocarbonyl)-4/5-carboxyphenyl-3H-xanthene-3-one | IV & VIII: $R=R^2=R^3=R^5=R^6=H$; Y=COOH | Light brown solid | 112° to 116°C Decomposed | 3340 $cm^{-1}$ to 3100 $cm^{-1}$ (N-H;s) 1700 $cm^{-1}$ (C=O;s) | Consistent | Red |
| 37 | 1.25 g of a Mixture of 2-amino-2',4',5',7'-tetraiodo-4,5,6,7-tetrachloro-3',6'-dihydroxy-spiro-[1H-isoindole-1,9'-[9H]-xanthene]-3(2H)-one and 6-hydroxy-2,4,5,7-tetraiodo-9-(2-hydrazocarbonyl-3,4,5,6-tetrachlorophenyl)-3H-xanthene-3-one | IV & VIII: $R=R^2=R^3=R^5=I$; $Y=Cl_4$ | Light orange solid | 208° to 213°C Decomposed | 3340 $cm^{-1}$ to 3180 $cm^{-1}$ (N-H;m) 1685 $cm^{-1}$ (C=O;s) | Consistent | Orange |

It is contemplated that by following the procedure described in the foregoing examples but employing the appropriate 2-R-3-$R^1$-6-(N-$R^9$-N-$R^{10}$-amino)-5'/6'-Y-fluoran with the appropriate $R^6$-substituted hydrazine of Formula X, there will be obtained 2-(N-$R^6$-amino)-2'-R-3'-$R^1$-6'-(N-$R^9$-N-$R^{10}$-amino)-5/6-Y-spiro[isoindole-1,9'-[9H]-xanthene]-3(2H)-one of Formula II, presented in Examples 38 to 46, presented in Table C hereinbelow.

TABLE C

| Example No. | R | $R^1$ | $R^6$ | $R^9$ | $R^{10}$ | Y |
|---|---|---|---|---|---|---|
| 38 | H | $C_4H_9$ | $2-ClC_6H_4$ | $C_6H_{11}$ | $CH_3$ | H |
| 39 | Cl | H | $4-CH_3OC_6H_4$ | $C_2H_5$ | $C_2H_5$ | $C_8H_{17}COO$ |
| 40 | $CH_3$ | $CH_3$ | $4-Cl-2-CH_3C_6H_3$ | $C_6H_5$ | $C_2H_5$ | $NO_2$ |
| 41 | Br | $C_2H_5$ | $3-NO_2C_6H_4$ | $C_4H_9$ | $C_4H_9$ | H |
| 42 | $C_2H_5$ | H | H | $2,4-(CH_3)_2C_6H_3$ | $CH_3$ | $NO_2$ |
| 43 | $C_4H_9$ | H | $CH_3$ | $4-ClC_6H_4CH_2$ | H | $C_4H_9COO$ |
| 44 | I | $C_3H_7$ | $4-BrC_6H_4$ | $C_6H_4CH_2$ | $C_6H_4CH_2$ | H |
| 45 | $C_2H_5$ | $C_2H_5$ | H | $4-CH_3OC_6H_4$ | $CH_3$ | H |
| 46 | Cl | $CH_3$ | $C_6H_5$ | $4-ClC_6H_4CH_2$ | $C_2H_5$ | $C_{12}H_{25}COO$ |

It is contemplated that by following the procedure described in the foregoing examples but employing the appropriate 2-(N-$R^7$-N-$R^8$-amino)-3-$R^1$-6-(N-$R^9$-N-$R^{10}$-amino)-5'/6'-Y-fluoran with the appropriate $R^6$-substituted hydrazine of Formula X, there will be obtained 2-(N-$R^6$-amino)-2'-(N-$R^7$-N-$R^8$-amino)-3'-$R^1$-6'-(N-$R^9$-N-$R^{10}$-amino)-5/6-Y-spiro[1H-isoindole-1,9'-[9H]-xanthene]-3(2H)-one of Formula III, presented in Examples 47 to 57, presented in Table D hereinbelow.

TABLE D

| Example No. | $R^1$ | $R^6$ | $R^7$ | $R^8$ | $R^9$ | $R^{10}$ | Y |
|---|---|---|---|---|---|---|---|
| 47 | H | $3\text{-}NO_2C_6H_4$ | $C_4H_9$ | $C_4H_9$ | $C_2H_5$ | $2,4\text{-}ClC_6H_3CH_2$ | H |
| 48 | $CH_3$ | $CH_3$ | $4\text{-}CH_3C_6H_4CH_2$ | $4\text{-}CH_3C_6H_4CH_2$ | $2,4\text{-}(CH_3)_2C_6H_3$ | $CH_3$ | $NO_2$ |
| 49 | $C_4H_9$ | $4\text{-}BrC_6H_4$ | $CH_3$ | $2,4\text{-}Cl_2C_6H_3$ | $4\text{-}ClC_6H_4$ | $C_4H_9$ | $C_4H_9COO$ |
| 50 | $C_2H_5$ | $2\text{-}CH_3C_6H_4$ | $C_{12}H_{25}$ | $C_{12}H_{25}$ | $C_6H_{11}$ | $C_4H_9$ | H |
| 51 | $CH_3$ | H | $2,4\text{-}Cl_2C_6H_3CH_2$ | $2,4\text{-}Cl_2C_6H_3CH_2$ | $C_4H_9$ | $C_4H_9$ | H |
| 52 | $C_3H_7$ | $C_2H_5$ | $C_2H_5$ | $4\text{-}CH_3C_6H_4$ | $4\text{-}NO_2C_6H_4CH_2$ | $CH_3$ | $NO_2$ |
| 53 | H | $4\text{-}CH_3C_6H_4$ | $CH_3$ | $4\text{-}BrC_6H_4$ | $2,4\text{-}(CH_3O)_2C_6H_3CH_2$ | $CH_3$ | H |
| 54 | $C_4H_9$ | H | $CH_3CO$ | $2\text{-}ClC_6H_4CH_2$ | $C_6H_5$ | $C_3H_7$ | $C_{12}H_{25}COO$ |
| 55 | $CH_3$ | $C_6H_5$ | $C_8H_{17}$ | $C_8H_{17}$ | $4\text{-}BrC_6H_4CH_2$ | $4\text{-}BrC_6H_4CH_2$ | H |
| 56 | H | $CH_3$ | $4\text{-}CH_3OC_6H_4CH_2$ | $4\text{-}CH_3OC_6H_4CH_2$ | $C_2H_5$ | $C_2H_5$ | $C_2H_5COO$ |
| 57 | $C_2H_5$ | H | $C_3H_7$ | $C_3H_7$ | $2,4\text{-}Cl_2C_6H_3$ | $CH_3$ | H |

It is contemplated that by following the procedure described in the foregoing examples but employing the appropriate 2-R-3,6-dihydroxy-4-$R^2$-5-$R^3$-7-$R^5$-5'/6'-Y-fluoran with the appropriate $R^6$-substituted hydrazine of Formula X, there will be obtained 2-(N-$R^6$-amino)-2'-R-3',6'-dihydroxy-4'-$R^2$-5'-$R^3$-7'-$R^5$-5/6-Y-spiro[1H-isoindole-1,9'-[9H]-xanthene]-3(2H)-one of Formula IV, presented in Examples 58 to 67, presented in Table E hereinbelow.

TABLE E

| Example No. | R | $R^2$ | $R^3$ | $R^5$ | $R^6$ | Y |
|---|---|---|---|---|---|---|
| 58 | H | Br | Br | Br | $CH_3$ | H |
| 59 | Cl | Cl | Cl | Cl | H | H |
| 60 | H | Cl | Cl | H | $C_6H_5$ | $C_2H_5COO$ |
| 61 | I | I | I | I | H | H |
| 62 | H | H | H | Br | $2,4-(CH_3)_2C_6H_3$ | $NO_2$ |
| 63 | Br | Br | Br | Br | H | $C_8H_{17}COO$ |
| 64 | H | F | F | H | $4-CH_3C_6H_4$ | $C_4H_9COO$ |
| 65 | Cl | Cl | Cl | Cl | $2,5-(Cl)_2C_6H_3$ | H |
| 66 | H | Br | Br | H | $2-NO_2C_6H_4$ | $C_2H_5COO$ |
| 67 | Br | Br | Br | Br | $CH_3$ | H |

It is contemplated that by following the procedure described in the foregoing examples but employing the appropriate 2-R-3-$R^1$-4-$R^2$-6-(N-$R^9$-N-$R^{10}$-amino)-5'/6'-Y-fluoran with the appropriate $R^6$-substituted hydrazine of Formula X, there will be obtained 3-$R^6$-2'-R-3'-$R^1$-6'-(N-$R^9$-N-$R^{10}$-amino)-6/7-Y-2,3-dihydro-spiro[phthalazine-1(4H),9'-[9H]-xanthene]-4-one of Formula VI, presented in Examples 68 to 76, presented in Table F hereinbelow.

TABLE F

| Example No. | R | $R^1$ | $R^6$ | $R^9$ | $R^{10}$ | Y |
|---|---|---|---|---|---|---|
| 68 | H | $C_4H_9$ | $2\text{-}ClC_6H_4$ | $C_6H_{11}$ | $CH_3$ | H |
| 69 | Cl | H | $4\text{-}CH_3OC_6H_4$ | $C_2H_5$ | $C_2H_5$ | $C_8H_{17}COO$ |
| 70 | $CH_3$ | $CH_3$ | $4\text{-}Cl\text{-}2\text{-}CH_3C_6H_3$ | $C_6H_5$ | $C_2H_5$ | $NO_2$ |
| 71 | Br | $C_2H_5$ | $3\text{-}NO_2C_6H_4$ | $C_4H_9$ | $C_4H_9$ | H |
| 72 | $C_2H_5$ | H | H | $2,4\text{-}(CH_3)_2C_6H_3$ | $CH_3$ | $NO_2$ |
| 73 | $C_4H_9$ | H | $CH_3$ | $4\text{-}ClC_6H_4CH_2$ | H | $C_4H_9COO$ |
| 74 | I | $C_3H_7$ | $4\text{-}BrC_6H_4$ | $C_6H_4CH_2$ | $C_6H_4CH_2$ | H |
| 75 | $C_2H_5$ | $C_2H_5$ | H | $4\text{-}CH_3OC_6H_4$ | $CH_3$ | H |
| 76 | Cl | $CH_3$ | $C_6H_5$ | $4\text{-}ClC_6H_4CH_2$ | $C_2H_5$ | $C_{12}H_{25}COO$ |

It is contemplated that by following the procedure described in the foregoing examples but employing the appropriate 2-(N-$R^7$-N-$R^8$-amino)-3-$R^1$-6-(N-$R^9$-N-$R^{10}$-amino)-5'/6'-Y-fluoran with the appropriate $R^6$-substituted hydrazine of Formula X, there will be obtained 3-$R^6$-2'-(N-$R^7$-N-$R^8$-amino)-3'-$R^1$-6'-(N-$R^9$-N-$R^{10}$-amino)-6/7-Y-2,3-dihydrospiro[phthalazine-1(4H),9'-[9H]-xanthene]-4-one of Formula VII, presented in Examples 77 to 87, presented in Table G hereinbelow.

TABLE G

| Example No. | $R^1$ | $R^6$ | $R^7$ | $R^8$ | $R^9$ | $R^{10}$ | Y |
|---|---|---|---|---|---|---|---|
| 77 | H | $3-NO_2C_6H_4$ | $C_4H_9$ | $C_4H_9$ | $C_2H_5$ | $2,4-ClC_6H_3CH_2$ | H |
| 78 | $CH_3$ | $CH_3$ | $4-CH_3C_6H_4CH_2$ | $4-CH_3C_6H_4CH_2$ | $2,4-(CH_3)_2C_6H_3$ | $CH_3$ | $NO_2$ |
| 79 | $C_4H_9$ | $4-BrC_6H_4$ | $CH_3$ | $2,4-Cl_2C_6H_3$ | $4-ClC_6H_4$ | $C_4H_9$ | $C_4H_9COO$ |
| 80 | $C_2H_5$ | $2-CH_3C_6H_4$ | $C_{12}H_{25}$ | $C_{12}H_{25}$ | $C_6H_{11}$ | $C_4H_9$ | H |
| 81 | $CH_3$ | H | $2,4-Cl_2C_6H_3CH_2$ | $2,4-Cl_2C_6H_3CH_2$ | $C_4H_9$ | $C_4H_9$ | H |
| 82 | $C_3H_7$ | $C_2H_5$ | $C_2H_5$ | $4-CH_3C_6H_4$ | $4-NO_2C_6H_4CH_2$ | $CH_3$ | $NO_2$ |
| 83 | H | $4-CH_3C_6H_4$ | $CH_3$ | $4-BrC_6H_4$ | $2,4-(CH_3O)_2C_6H_3CH_2$ | $CH_3$ | H |
| 84 | $C_4H_9$ | H | $CH_3CO$ | $2-ClC_6H_4CH_2$ | $C_6H_5$ | $C_3H_7$ | $C_{12}H_{25}COO$ |
| 85 | $CH_3$ | $C_6H_5$ | $C_8H_{17}$ | $C_8H_{17}$ | $4-BrC_6H_4CH_2$ | $4-BrC_6H_4CH_2$ | H |
| 86 | H | $CH_3$ | $4-CH_3OC_6H_4CH_2$ | $4-CH_3OC_6H_4CH_2$ | $C_2H_5$ | $C_2H_5$ | $C_2H_5COO$ |
| 87 | $C_2H_5$ | H | $C_3H_7$ | $C_3H_7$ | $2,4-Cl_2C_6H_3$ | $CH_3$ | H |

It is contemplated that by following the procedure described in the foregoing examples but employing 2-R-3,6-dihydroxy-4-$R^2$-5-$R^3$-7-$R^5$-5'/6'-Y-fluoran with the appropriate $R^6$-substituted hydrazine of Formula X, there will be obtained 2-R-4-$R^2$-5-$R^3$-6-hydroxy-7-$R^5$-9-[2-(N-$R^6$-hydrazocarbonyl)-Y-phenyl]-3H-xanthene-3-one of Formula VIII, presented in Examples 88 to 97, presented in Table H hereinbelow.

TABLE H

| Example No. | R | $R^2$ | $R^3$ | $R^5$ | $R^6$ | Y |
|---|---|---|---|---|---|---|
| 88 | H | Br | Br | Br | $CH_3$ | H |
| 89 | Cl | Cl | Cl | Cl | H | H |
| 90 | H | Cl | Cl | H | $C_6H_5$ | $C_2H_5COO$ |
| 91 | I | I | I | I | H | H |
| 92 | H | H | H | Br | 2,4-$(CH_3)_2C_6H_3$ | $NO_2$ |
| 93 | Br | Br | Br | Br | H | $C_8H_{17}COO$ |
| 94 | H | F | F | H | 4-$CH_3C_6H_4$ | $C_4H_9COO$ |
| 95 | Cl | Cl | Cl | Cl | 2,5-$(Cl)_2C_6H_3$ | H |
| 96 | H | Br | Br | H | 2-$NO_2C_6H_4$ | $C_2H_5COO$ |
| 97 | Br | Br | Br | Br | $CH_3$ | H |

The compounds of Formulas I, V and VIII hereinabove are essentially colorless in the depicted form. When contacted with an electric current from an applied voltage stylus of the type ordinarily employed in electrochromic recording systems, the compounds of Formulas I, V and VIII develop red, green, blue-green, blue, purple, magenta, gray, and black-colored images. These developed images are very insensitive to light, that is, once the color is developed it remains unchanged when subjected to light exposure. The developed images also possess excellent xerographic reproducibility.

The compounds of this invention may be incorporated in any of the commercially-accepted systems known in the electrochromic recording art. Typical techniques for the application of the color formers to paper are well-known and are described in numerous patents, for example, U.S. Patents Re. 29,427; 3,726,769; 3,864,684; 3,871,972; 3,951,-757; 4,017,366; and 4,133,933. In general, these references describe paper coatings which consist of the color-forming component, an organic metal salt, a binder and some type of conductor, either an inorganic salt or a conductive polymer. This mixture is milled together optionally in the presence of a non-ionic surface active agent until the desired particle size is obtained and then the mixture is coated on paper and dried. Optionally, the color-forming substance can be milled in the presence of a binder and the remaining components milled also in the presence of a binder and the two mixtures combined together prior to coating on paper.

Normally the surface of the coated paper is wet with a conductive solution containing an inorganic alkali metal or alkaline earth metal salt, for example, potassium chloride, calcium chloride, sodium chloride, sodium bromide, potassium bromide, potassium nitrate or sodium sulfate immediately prior to the printing with the applied voltage stylus. For a quick qualitative test, it has been determined that the color-forming component can be dissolved in a suitable volatile organic solvent, coated on paper and the coated paper dried to obtain a paper sheet coated with the color-forming component. This coated sheet can then be wet with a conductive salt solution and an image traced with an applied voltage stylus to develop the colored image.

The compounds of Formulas I, V and VIII can be used alone as color-forming components in electrochromic recording paper, can be used in admixture, or in admixture with one or more other color-forming compounds selected from the classes consisting of phthalides, for example, Crystal Violet Lactone; fluorans, for example, 3-diethylamino-5,7-dimethylfluoran; redox indicators, for example, phenothiazines such as benzoyl leuco methylene blue and various other types of color-forming components currently employed in commercially-available electrochromic recording systems.

# CLAIMS

1. A compound which is a 2-($R^6$-amino)-2'-R-3'-$R^1$-4'-$R^2$-5'=$R^3$-6'-$R^4$-7'-$R^5$-5/6-Y-spiro[1H-isoindole-1,9'-[9H]-zanthene]-3(2H)-one having the structural formula

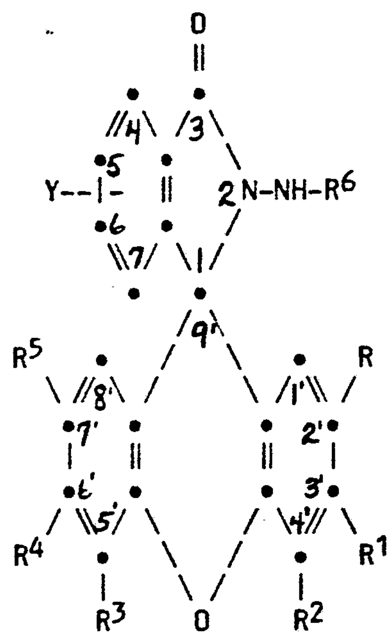

wherein:

R represents hydrogen, halo, non-tertiary $C_1$ to $C_4$ alkyl or $-N(R^7)(R^8)$ in which

$R^7$ represents hydrogen, non-tertiary $C_1$ to $C_{12}$ alkyl, acetyl, benzyl or benzyl substituted in the benzene ring by one or two of halo, nitro, non-tertiary $C_1$ to $C_4$ alkyl or non-tertiary $C_1$ to $C_4$ alkoxy, and

$R^8$ represents hydrogen, non-tertiary $C_1$ to $C_{12}$ alkyl, phenyl, phenyl substituted by one or two of halo, nitro,

non-tertiary $C_1$ to $C_4$ alkyl or non-tertiary $C_1$ to $C_4$ alkoxy, benzyl, benzyl substituted in the benzene ring by one or two of halo, nitro, non-tertiary $C_1$ to $C_4$ alkyl or non-tertiary $C_1$ to $C_4$ alkoxy, the group

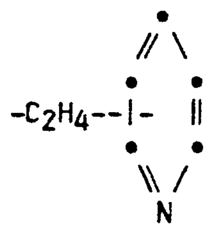

or

$R^7$ and $R^8$, taken together with the nitrogen, represent pyrrolyl;

$R^1$ represents hydrogen, hydroxy or non-tertiary $C_1$ to $C_4$ alkyl;

$R^2$, $R^3$ and $R^5$ independently represent hydrogen or halo;

$R^4$ represents hydroxy or $-N(R^9)(R^{10})$ in which

$R^9$ represents non-tertiary $C_1$ to $C_4$ alkyl, cyclohexyl, phenyl, phenyl substituted by one or two of halo, nitro, non-tertiary $C_1$ to $C_4$ alkyl or non-tertiary $C_1$ to $C_4$ alkoxy, benzyl, or benzyl substituted in the benzene ring by one or two of halo, nitro, non-tertiary $C_1$ to $C_4$ alkyl or non-tertiary $C_1$ to $C_4$ alkoxy,

$R^{10}$ represents non-tertiary $C_1$ to $C_4$ alkyl, benzyl or benzyl substituted in the benzene ring by one or two of halo, nitro, non-tertiary $C_1$ to $C_4$ alkyl or non-tertiary $C_1$ to $C_4$ alkoxy,or

$R^9$ and $R^{10}$, taken together with the nitrogen, represent pyrrolidyl or piperidyl;

$R^6$ represents hydrogen, non-tertiary $C_1$ to $C_4$ alkyl, phenyl, phenyl substituted by one or two of non-tertiary $C_1$ to $C_4$ alkyl, non-tertiary $C_1$ to $C_4$ alkoxy, halo, nitro or amino or $\overset{O}{\overset{\|}{C}}X$ in which X is non-tertiary $C_1$ to $C_4$ alkyl, non-tertiary $C_1$ to $C_4$ alkoxy, $NHNH_2$ or $\overset{O}{\overset{\|}{C}}NHNH_2$; and

Y represents hydrogen, nitro, amino, one to four halo, or $\overset{O}{\overset{\|}{C}}Z$ in which Z represents $OR^{11}$ wherein $R^{11}$ represents hydrogen, a non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy.

2. A compound according to claim 1, wherein $R^2$, $R^3$ and $R^5$ are hydrogen and $R^4$ is $-N(R^9)(R^{10})$.

3. A compound according to claim 1, wherein $R^2$, $R^3$ and $R^5$ are hydrogen, R is $-N(R^7)(R^8)$ and $R^4$ is $-N(R^9)(R^{10})$.

4. A 2-$R^6$-2'-R-3'-$R^1$-4'-$R^2$-5'-$R^3$-6'-$R^4$-7'-$R^5$-5/6-Y-2,3-dihydro-spiro[phthalazine-1(4H),9'-[9H]-xanthene]-4-one having the structural formula

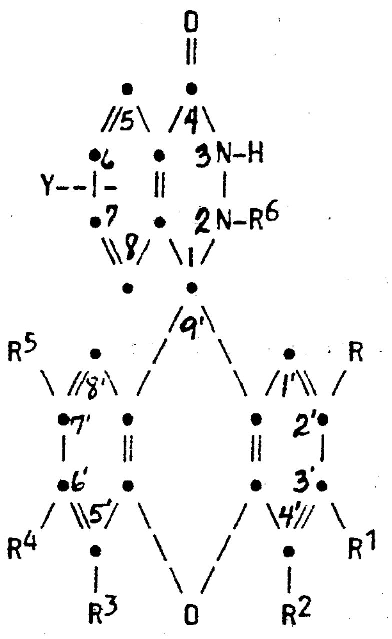

wherein:

R represents hydrogen, halo, non-tertiary $C_1$ to $C_4$ alkyl or $-N(R^7)(R^8)$ in which

$R^7$ represents hydrogen, non-tertiary $C_1$ to $C_{12}$ alkyl, acetyl, benzyl or benzyl substituted in the benzene ring by one or two of halo, nitro, non-tertiary $C_1$ to $C_4$ alkyl or non-tertiary $C_1$ to $C_4$ alkoxy, and

$R^8$ represents hydrogen, non-tertiary $C_1$ to $C_{12}$ alkyl, phenyl, phenyl substituted by one or two of halo, nitro, non-tertiary $C_1$ to $C_4$ alkyl or non-tertiary $C_1$ to $C_4$ alkoxy, benzyl, benzyl substituted in the benzene rihg by one or two of halo, nitro, non-terti-

ary $C_1$ to $C_4$ alkyl or non-tertiary $C_1$ to $C_4$ alkoxy, the group

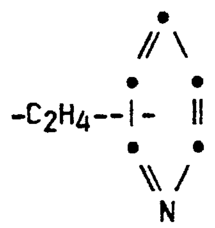

or

$R^7$ and $R^8$, taken together with the nitrogen, represent pyrrolyl;

$R^1$ represents hydrogen, hydroxy or non-tertiary $C_1$ to $C_4$ alkyl;

$R^2$, $R^3$ and $R^5$ independently represent hydrogen or halo;

$R^4$ represents hydroxy or $-N(R^9)(R^{10})$ in which

$R^9$ represents non-tertiary $C_1$ to $C_4$ alkyl, cyclohexyl, phenyl, phenyl substituted by one or two of halo, nitro, non-tertiary $C_1$ to $C_4$ alkyl or non-tertiary $C_1$ to $C_4$ alkoxy, benzyl, or benzyl substituted in the benzene ring by one or two of halo, nitro, non-tertiary $C_1$ to $C_4$ alkyl or non-tertiary $C_1$ to $C_4$ alkoxy, and

$R^{10}$ represents non-tertiary $C_1$ to $C_4$ alkyl, benzyl or benzyl substituted in the benzene ring by one or two of halo, nitro, non-tertiary $C_1$ to $C_4$ alkyl or non-tertiary $C_1$ to $C_4$ alkoxy, or

$R^9$ and $R^{10}$, taken together with the nitrogen, represent pyrrolidyl or piperidyl;

$R^6$ represents hydrogen, non-tertiary $C_1$ to $C_4$ alkyl, phenyl, phenyl substituted by one or two of non-tertiary $C_1$ to $C_4$ alkyl, non-tertiary $C_1$ to $C_4$ alkoxy, halo, nitro or amino or $\overset{O}{\overset{\|}{C}}X$ in which X is non-tertiary $C_1$ to $C_4$ alkyl, non-tertiary $C_1$ to $C_4$ alkoxy, $NHNH_2$ or $\overset{O}{\overset{\|}{C}}NHNH_2$; and

Y represents hydrogen, nitro, amino, one to four halo, or $\overset{O}{\overset{\|}{C}}Z$ in which Z represents $OR^{11}$ wherein $R^{11}$ represents a non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy.

5. A compound according to claim 4, wherein $R^2$, $R^3$ and $R^5$ are hydrogen and $R^4$ is $-N(R^9)(R^{10})$.

6. A compound according to claim 4, wherein $R^2$, $R^3$ and $R^5$ are hydrogen, R is $-N(R^7)(R^8)$ and $R^4$ is $-N(R^9)(R^{10})$.

7. A 2-R-4-$R^2$-5-$R^3$-6-hydroxy-7-$R^5$-9-[2-(N-$R^6$-hydrazocarbonyl)-Y-phenyl]-[3H]-xanthene-3-one having the structural formula

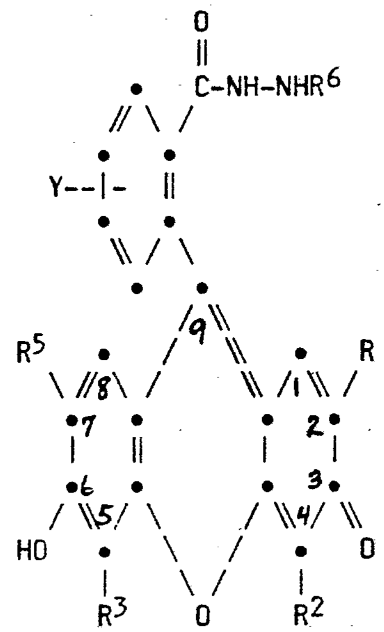

wherein:

R, $R^2$, $R^3$ and $R^5$ independently represent hydrogen or halo;

$R^6$ represents hydrogen, non-tertiary $C_1$ to $C_4$ alkyl, phenyl, phenyl substituted by one or two of non-tertiary $C_1$ to $C_4$ alkyl, non-tertiary $C_1$ to $C_4$ alkoxy, halo, nitro or amino or $\overset{O}{\overset{\|}{C}}X$ in which X is non-tertiary $C_1$ to $C_4$ alkyl, non-tertiary $C_1$ to $C_4$ alkoxy, $NHNH_2$ or $\overset{O}{\overset{\|}{C}}NHNH_2$; and

Y represents hydrogen, nitro, amino, one to four halo, or $\overset{O}{\overset{\|}{C}}Z$ in which Z represents $OR^{11}$ wherein $R^{11}$ represents a non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy.

8. A substrate for use in electrochromic recording comprising a support sheet containing as a color-forming substance a compound according to any one of claims 1-7.

9. A process for preparing a compound selected from the group having the structural formula

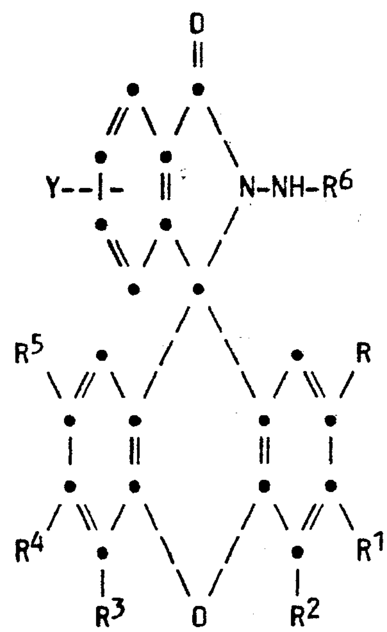

and the structural formula

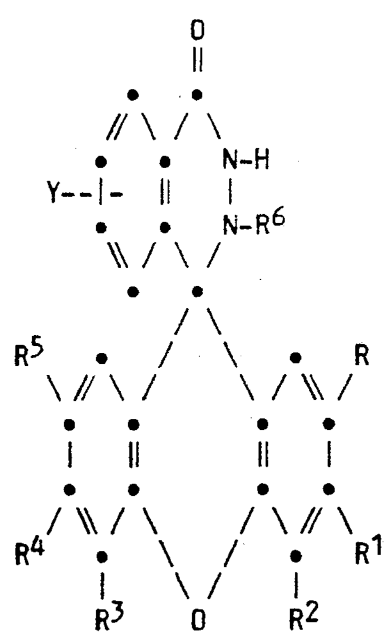

and mixtures thereof which comprises interacting a compound having the structural formula

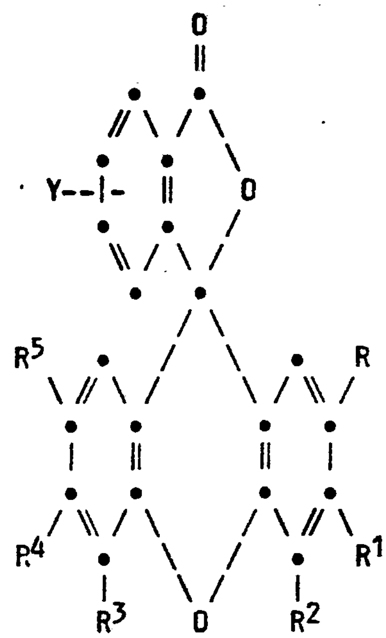

with a compound of the formula $R^6NHNH_2$ wherein:

R represents hydrogen, halo, non-tertiary $C_1$ to $C_4$ alkyl or $-N(R^7)(R^8)$ in which

$R^7$ represents hydrogen, non-tertiary $C_1$ to $C_{12}$ alkyl, acetyl, benzyl or benzyl substituted in the benzene ring by one or two of halo, nitro, non-tertiary $C_1$ to $C_4$ alkyl or non-tertiary $C_1$ to $C_4$ alkoxy, and

$R^8$ represents hydrogen, non-tertiary $C_1$ to $C_{12}$ alkyl, phenyl, phenyl substituted by one or two of halo, nitro, non-tertiary $C_1$ to $C_4$ alkyl or non-tertiary $C_1$ to $C_4$ alkoxy, benzyl, benzyl substituted in the benzene ring by one or two of halo, nitro, non-tertiary $C_1$ to $C_4$ alkyl or non-tertiary $C_1$ to $C_4$ alkoxy, the group

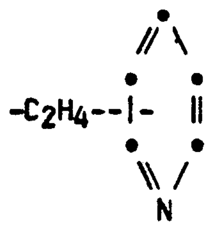 or

$R^7$ and $R^8$, taken together with the nitrogen, represent pyrrolyl;

$R^1$ represents hydrogen, hydroxy or non-tertiary $C_1$ to $C_4$ alkyl;

$R^2$, $R^3$ and $R^5$ independently represent hydrogen or halo;

$R^4$ represents hydroxy or $-N(R^9)(R^{10})$ in which

$R^9$ represents non-tertiary $C_1$ to $C_4$ alkyl, cyclohexyl, phenyl, phenyl substituted by one or two of halo, nitro, non-tertiary $C_1$ to $C_4$ alkyl or non-tertiary $C_1$ to $C_4$ alkoxy, benzyl, or benzyl substituted in the benzene ring by one or two of halo, nitro, non-tertiary $C_1$ to $C_4$ alkyl or non-tertiary $C_1$ to $C_4$ alkoxy,

$R^{10}$ represents non-tertiary $C_1$ to $C_4$ alkyl, benzyl or benzyl substituted in the benzene ring by one or two of halo, nitro, non-tertiary $C_1$ to $C_4$ alkyl or non-tertiary $C_1$ to $C_4$ alkoxy,

$R^9$ and $R^{10}$, taken together with the nitrogen, represent pyrrolidyl or piperidyl;

$R^6$ represents hydrogen, non-tertiary $C_1$ to $C_4$ alkyl, phenyl, phenyl substituted by one or two of non-tertiary $C_1$ to $C_4$ alkyl, non-tertiary $C_1$ to $C_4$ alkoxy, halo, nitro or amino or $\overset{O}{\overset{\parallel}{C}}X$ in which X is non-tertiary $C_1$ to $C_4$ alkyl, non-tertiary $C_1$ to $C_4$ alkoxy, $NHNH_2$ or $\overset{O}{\overset{\parallel}{C}}NHNH_2$; and

Y represents hydrogen, nitro, amino, one to four halo, or $\overset{O}{\overset{\parallel}{C}}Z$ in which Z represents $OR^{11}$ wherein $R^{11}$ represents hydrogen, a non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy

10. A process for preparing a compound selected from the group having the structural formula

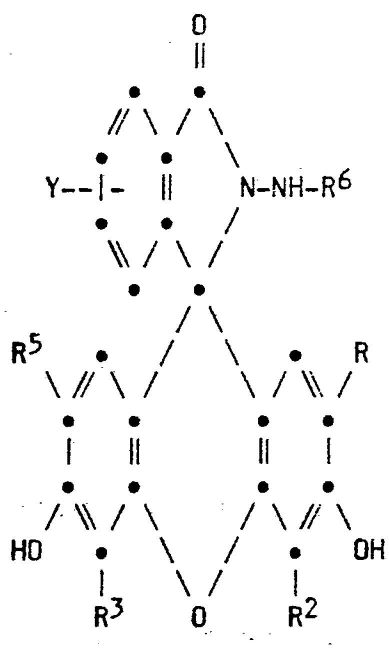

and the structural formula

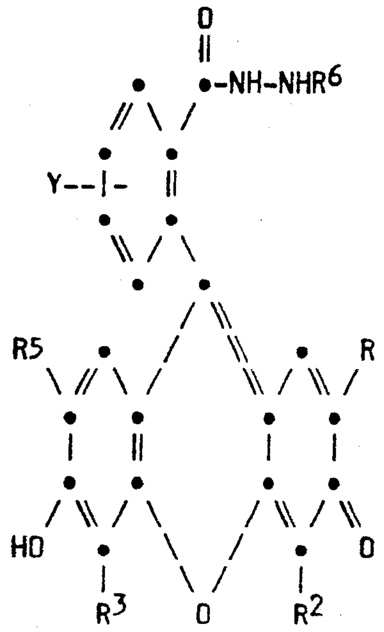

$R^1 = O$

$R^4 = OH$

and mixtures thereof which comprises interacting a compound having the structural formula

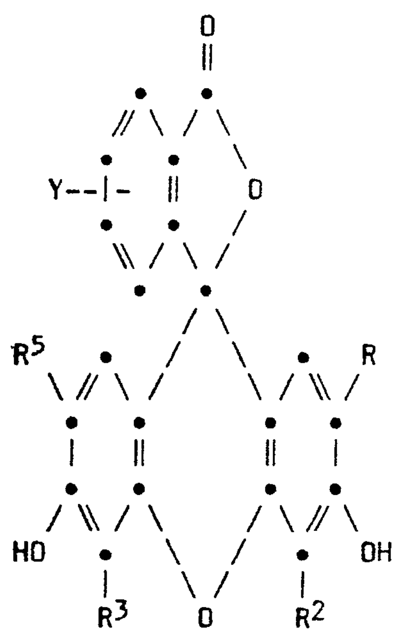

with a compound of the formula $R^6NHNH_2$ wherein:

R, $R^2$, $R^3$ and $R^5$ independently represent hydrogen or halo;

$R^6$ represents hydrogen, non-tertiary $C_1$ to $C_4$ alkyl, phenyl, phenyl substituted by one or two of non-tertiary $C_1$ to $C_4$ alkyl, non-tertiary $C_1$ to $C_4$ alkoxy, halo, nitro or amino or $\overset{\overset{O}{\|}}{C}X$ in which X is non-tertiary $C_1$ to $C_4$ alkyl, non-tertiary $C_1$ to $C_4$ alkoxy, $NHNH_2$ or $\overset{\overset{O}{\|}}{C}NHNH_2$; and

Y represents hydrogen, nitro, amino, one to four halo, or $\overset{\overset{O}{\|}}{C}Z$ in which Z represents $OR^{11}$ wherein $R^{11}$ represents a non-tertiary $C_1$ to $C_{16}$ alkyl, benzyl or benzyl substituted in the benzene ring by one or two of halo, nitro, non-tertiary $C_1$ to $C_4$ alkyl or non-tertiary $C_1$ to $C_4$ alkoxy.

European Patent Office

# EUROPEAN SEARCH REPORT

Application number: EP 84110835.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 4 268 514 (R. GALT; R. PEARCE)<br>* Claim 1 *<br>-- | 1,4 | C 07 D 491/107<br>C 07 D 311/82<br>G 03 C 65/12 |
| A | US - A - 4 017 366 (P. HSIEH; E. ROGALL)<br>* Claims; column 4, lines 47-49 *<br>-- | 8 | |
| A | US - A - 4 304 720 (DEAN et al.)<br>* Claim 1 *<br>-- | 7 | |
| A | US - A - 4 290 955 (CINCOTTA et al.)<br>* Abstract *<br>---- | 7 | |

TECHNICAL FIELDS SEARCHED (Int. Cl.4)

C 07 D 491/00
G 03 G 5/00
C 07 D 311/00

The present search report has been drawn up for all claims

| Place of search | Date of completion of the search | Examiner |
|---|---|---|
| VIENNA | 11-12-1984 | BRUS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document